(19) Europäisches Patentamt
European Patent Office
Office européen des brevets

(11) EP 1 100 775 B1

(12) **EUROPÄISCHE PATENTSCHRIFT**

(45) Veröffentlichungstag und Bekanntmachung des Hinweises auf die Patenterteilung:
**17.12.2003 Patentblatt 2003/51**

(21) Anmeldenummer: **99934567.1**

(22) Anmeldetag: **03.07.1999**

(51) Int Cl.⁷: **C07C 311/00**

(86) Internationale Anmeldenummer:
**PCT/EP99/04643**

(87) Internationale Veröffentlichungsnummer:
**WO 00/003978 (27.01.2000 Gazette 2000/04)**

(54) **2,5-SUBSTITUIERTE BENZOLSULFONYLHARNSTOFFE UND -THIOHARNSTOFFE, VERFAHREN ZU IHRER HERSTELLUNG, IHRE VERWENDUNG UND SIE ENTHALTENDE PHARMZEUTISCHE PRÄPARATE**

2,5-SUBSTITUTED BENZOLSULFONYLUREAS AND THIOUREAS, METHODS FOR THE PRODUCTION THEREOF, USE THEREOF AND PHARMACEUTICAL PREPARATIONS CONTAINING THE SAME

BENZOLSULFONYL-UREES ET -THIOUREES SUBSTITUEES EN 2,5, LEUR PROCEDE DE PRODUCTION, LEUR UTILISATION ET LES PREPARATIONS PHARMACEUTIQUES LES CONTENANT

(84) Benannte Vertragsstaaten:
**AT BE CH CY DE DK ES FI FR GB GR IE IT LI LU MC NL PT SE**

(30) Priorität: **16.07.1998 DE 19832009**

(43) Veröffentlichungstag der Anmeldung:
**23.05.2001 Patentblatt 2001/21**

(73) Patentinhaber: **Aventis Pharma Deutschland GmbH**
**65929 Frankfurt am Main (DE)**

(72) Erfinder:
- **HEITSCH, Holger**
  **D-55252 Mainz-Kastel (DE)**
- **ENGLERT, Heinrich, Christian**
  **D-65719 Hofheim (DE)**
- **GÖGELEIN, Heinz**
  **D-60528 Frankfurt (DE)**

(56) Entgegenhaltungen:
**EP-A- 0 612 724** **EP-A- 0 657 423**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 100 775 B1

## Beschreibung

Die vorliegende Erfindung betrifft neue 2,5-substituierte Benzolsulfonylharnstoffe und -thioharnstoffe der Formel I,

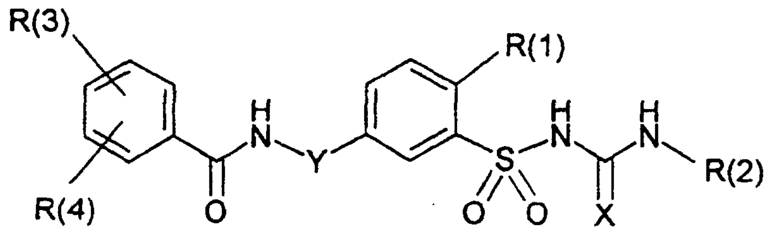

I

in der R(1 ), R(2), R(3), R(4), X und Y die unten angegebenen Bedeutungen haben, die wertvolle Arzneimittelwirkstoffe sind. Die Verbindungen der Formel I haben eine inhibierende Wirkung auf ATP-sensitive Kaliumkanäle und eignen sich zum Beispiel zur Behandlung von Störungen des Herz-Kreislaufsystems, insbesondere zur Behandlung von Arrhythmien, zur Verhinderung des plötzlichen Herztodes oder zur Beeinflussung einer verminderten Kontraktilität des Herzens. Die Erfindung betrifft weiterhin Verfahren zur Herstellung der Verbindungen der Formel I, ihre Verwendung und sie enthaltende pharmazeutische Präparate.

Für bestimmte Benzolsulfonylharnstoffe ist eine blutzuckersenkende Wirkung beschrieben. Als Prototyp solcher blutzuckersenkender Sulfonylharnstoffe gilt das Glibenclamid. das als Mittel zur Behandlung des Diabetes mellitus therapeutisch verwendet wird. Glibenclamid blockiert ATP-sensitive Kaliumkanäle und dient in der Forschung als Werkzeug zur Erforschung derartiger Kaliumkanäle. Neben seiner blutzuckersenkenden Wirkung besitzt das Glibenclamid noch andere Wirkungen, die auf die Blockade eben dieser ATP-sensitiven Kaliumkanäle zurückgeführt werden, die jedoch bislang therapeutisch noch nicht genutzt werden können. Dazu gehört insbesondere eine antifibrillatorische Wirkung am Herzen. Bei der Behandlung des

Kammerflimmerns oder seiner Vorstufen mit Glibenclamid wäre aber die durch diese Substanz gleichzeitig hervorgerufene Blutzuckersenkung unerwünscht oder gar gefährlich, da sie den Zustand des Patienten weiter verschlechtern kann.

Aus den Patentanmeldungen EP-A-612 724, EP-A-657 423, EP-A-661 264, EP-A-726 250, EP-A-727 416, EP-A-727 417 und EP-A-728 741 sind antifibrillatorische Benzolsulfonylhamstoffe und -thioharnstoffe mit verminderter blutzuckersenkender Wirkung bekannt. Die Eigenschaften dieser Verbindungen sind jedoch in verschiedener Hinsicht noch nicht befriedigend, und es besteht weiterhin Bedarf an Verbindungen mit einem günstigeren pharmakodynamischen bzw. pharmakokinetischen Eigenschaftsprofil, die insbesondere zur Behandlung eines gestörten Herzrhythmus und seiner Folgen besser geeignet sind. Überraschend wurde nun gefunden, daß sich bestimmte 2,5-substituierte Benzolsulfonylharnstoffe und -thioharnstoffe, die in der 2-Position einen ungesättigten Rest enthalten, durch eine ausgeprägte Wirkung auf ATP-sensitive Kaliumkanäle auszeichnen.

Gegenstand der vorliegenden Erfindung sind somit Verbindungen der Formel I,

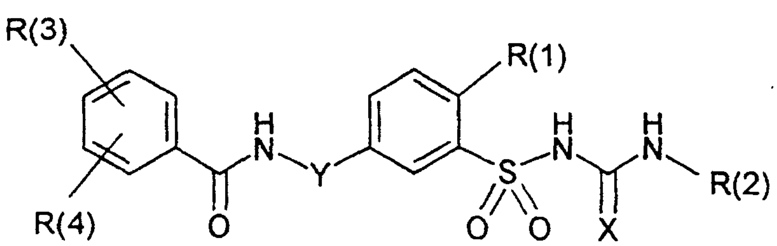

I

in der

X für Sauerstoff oder Schwefel steht;

Y für $-(CR(5)_2)_n-$ steht;

R(1) für

1. Phenyl, das unsubstituiert ist oder substituiert ist durch einen oder zwei gleiche oder verschiedene Substituenten aus der Reihe Halogen, $(C_1-C_4)$-Alkyl, $(C_1-C_4)$-Alkoxy, $-S(O)_m-(C_1-C_4)$-Alkyl, Phenyl, Amino, Hydroxy, Nitro, Trifluormethyl, Cyan, Hydroxycarbonyl, Carbamoyl, $(C_1-C_4)$-Alkoxycarbonyl und Formyl; oder
2. Naphthyl; oder
3. monocyclisches oder bicyclisches Heteroaryl mit ein oder zwei gleichen oder verschiedenen Ring-Heteroatomen aus der Reihe Sauerstoff, Schwefel und Stickstoff; oder
4. $-S(O)_m$-Phenyl; oder
5. $(C_2-C_5)$-Alkenyl, das unsubstituiert ist oder substituiert ist durch einen Rest aus der Reihe Phenyl, Cyan, Hydroxycarbonyl und $(C_1-C_4)$-Alkoxycarbonyl; oder
6. $(C_2-C_5)$-Alkinyl, das unsubstituiert ist oder substituiert ist durch einen Rest aus der Reihe Phenyl und $(C_1-C_4)$ -Alkoxy; steht;

R(2) für Wasserstoff oder $(C_1-C_3)$-Alkyl steht;
R(3) und R(4) unabhängig voneinander für Wasserstoff, Halogen oder $(C_1-C_4)$-Alkoxy stehen;
die Reste R(5), die alle unabhängig voneinander sind und gleich oder verschieden sein können, für Wasserstoff oder $(C_1-C_3)$-Alkyl stehen;
m für 0, 1 oder 2 steht;
n für 1, 2, 3 oder 4 steht;
in allen ihren stereoisomeren Formen und Mischungen davon in allen Verhältnissen, und ihre physiologisch verträglichen Salze.

Können Reste, Gruppen, Substituenten oder Variable mehrfach in den Verbindungen der Formel I vorkommen, so können sie alle unabhängig voneinander die angegebenen Bedeutungen haben und können jeweils gleich oder verschieden sein.

Der Begriff Alkyl bedeutet, sofern nicht anders angegeben, geradkettige oder verzweigte gesättigte Kohlenwasserstoffreste. Dies gilt auch für davon abgeleitete Reste wie zum Beispiel Alkoxy, Alkoxycarbonyl oder $-S(O)_m$-Alkyl. Beispiele für Alkylreste sind Methyl, Ethyl, n-Propyl, Isopropyl, n-Butyl, sec-Butyl, Isobutyl oder tert-Butyl. Beispiele für Alkoxy sind Methoxy, Ethoxy, n-Propoxy oder Isopropoxy.

Alkenyl und Alkinyl stehen für geradkettige oder verzweigte, einfach oder mehrfach ungesättigte Kohlenwasserstoffreste, in denen sich die Doppelbindungen und/oder Dreifachbindungen in beliebigen Positionen befinden können. Beispiele für Alkenyl und Alkinyl sind Vinyl, Prop-2-enyl (Allyl), Prop-1-enyl, Butenyl, 3-Methyl-but-2-enyl, Ethinyl, Prop-2-inyl (Propargyl), Prop-1-inyl, But-2-inyl und But-3-inyl.

Halogen steht für Fluor, Chlor, Brom oder lod, vorzugsweise für Chlor oder Fluor.

In substituierten Phenylresten können sich die Substituenten in beliebigen Positionen befinden. In monosubstituierten Phenylresten kann sich der Substituent in der 2-Position, der 3-Position oder der 4-Position befinden, in disubstituierten Phenylresten können sich die Substituenten in 2,3-Position, 2,4-Position, 2,5-Position, 2,6-Position, 3,4-Position oder 3,5-Position befinden. Trägt ein Phenylrest einen weiteren Phenylrest als Substituenten, so kann auch dieser Phenylrest wiederum unsubstituiert sein oder durch einen oder zwei gleiche oder verschiedene derjenigen Reste substituiert sein, die als Substituenten am ersten Phenylrest aufgeführt sind, außer durch einen Phenylrest. Naphthyl kann 1-Naphthyl oder 2-Naphthyl sein.

Unter Heteroaryl werden Reste von monocyclischen oder bicyclischen aromatischen Ringsystemen verstanden, die im Falle der Monocyclen einen 5-Ring oder einen 6-Ring und im Falle der Bicyclen zwei kondensierte 5-Ringe, einen mit einem 5-Ring kondensierten 6-Ring oder zwei kondensierte 6-Ringe enthalten. Sie können aufgefaßt werden als von Cyclopentadienyl, Phenyl, Pentalenyl, Indenyl oder Naphthyl durch Ersatz einer oder zweier CH-Gruppen und/ oder $CH_2$-Gruppen durch S, O, N, NH (oder einen Substituenten tragendes N wie zum Beispiel $N-CH_3$) abgeleitete Reste, wobei das aromatische Ringsystem erhalten bleibt oder ein aromatisches Ringsystem ausgebildet wird. Neben den ein oder zwei Ring-Heteroatomen enthalten sie drei bis neun Ring-Kohlenstoffatome. Beispiele für Heteroaryl sind insbesondere Furyl, Thienyl, Pyrrolyl, Imidazolyl, Pyrazolyl, 1,3-Oxazolyl, 1,2-Oxazolyl, 1,3-Thiazolyl, 1,2-Thiazolyl, Pyridyl, Pyridazinyl, Pyrazinyl, Pyrimidyl, Indolyl, Benzofuranyl, Benzothiophenyl, Chinolyl, Isochinolyl oder Benzopyranyl. Ein Heteroarylrest kann über jedes geeignete Kohlenstoffatom gebunden sein. Beispielsweise kann ein Thienylrest als 2-Thienylrest oder 3-Thienylrest vorliegen, ein Furylrest als 2-Furylrest oder 3-Furylrest, ein Pyridylrest als 2-Pyridylrest, 3-Pyridylrest oder 4-Pyridylrest. Ein Rest, der sich vom 1,3-Thiazol oder vom Imidazol ableitet, kann über die 2-Position, die 4-Position oder die 5-Position gebunden sein. Geeignete Stickstoffheterocyclen können auch als N-Oxide oder als Quartärsalze mit einem von einer physiologisch verträglichen Säure abgeleiteten Anion als Gegenion vorliegen. Pyridylreste zum Beispiel können also als Pyridin-N-oxide vorliegen.

Die vorliegende Erfindung umfaßt alle stereoisomeren Formen der Verbindungen der Formel I. In den Verbindungen der Formel I enthaltene Asymmetriezentren können alle unabhängig voneinander die S-Konfiguration oder die R-Konfiguration aufweisen. Zur Erfindung gehören alle möglichen Enantiomeren und Diastereomeren, ebenso Mischungen von zwei oder mehr stereoisomeren Formen, zum Beispiel Mischungen von Enantiomeren und/oder Diastereomeren, in allen Verhältnissen. Enantiomere sind also in enantiomerenreiner Form, sowohl als linksdrehende als auch als rechtsdrehende Antipoden, in Form von Racematen und in Form von Mischungen der beiden Enantiomeren in allen Verhältnissen Gegenstand der Erfindung. Bei Vorliegen einer cis/trans-Isomerie sind sowohl die cis-Form als auch die trans-Form und Gemische dieser Formen in allen Verhältnissen Gegenstand der Erfindung. Die Herstellung von einzelnen Stereoisomeren kann gewünschtenfalls durch Auftrennung eines Gemisches nach üblichen Methoden, zum Beispiel durch Chromatographie oder Kristallisation, oder durch Verwendung von stereochemisch einheitlichen Ausgangssubstanzen bei der Synthese oder durch Anwendung von stereoselektiven Reaktionen erfolgen. Gegebenenfalls kann vor einer Trennung von Stereoisomeren eine Derivatisierung erfolgen. Die Trennung eines Stereoisomerengemisches kann auf der Stufe der Verbindungen der Formel I erfolgen oder auf der Stufe eines Zwischenprodukts im Verlaufe der Synthese. Die Erfindung umfaßt auch alle tautomeren Formen der Verbindungen der Formel I.

Physiologisch verträgliche Salze der Verbindungen der Formel I sind insbesondere nicht toxische Salze oder pharmazeutisch verwendbare Salze. Sie können anorganische oder organische Salzkomponenten enthalten (siehe auch Remington's Pharmaceutical Sciences, A. R. Gennaro (Ed.), Mack Publishing Co., 17. Auflage, Seite 1418 (1985)). Solche Salze lassen sich beispielsweise aus Verbindungen der Formel I und nicht toxischen anorganischen oder organischen Basen herstellen, beispielsweise geeigneten Alkalimetall- oder Erdalkalimetallverbindungen, wie Natriumhydroxid oder Kaliumhydroxid, oder Ammoniak oder organischen Aminoverbindungen oder Ammoniumhydroxiden. Umsetzungen von Verbindungen der Formel I mit Basen zur Herstellung der Salze werden im allgemeinen gemäß üblichen Vorgehensweisen in einem Lösungsmittel oder Verdünnungsmittel durchgeführt. Aufgrund der physiologischen und chemischen Stabilität vorteilhafte Salze sind beim Vorliegen saurer Gruppen in vielen Fällen Natrium-, Kalium-, Magnesium- oder Calciumsalze oder Ammoniumsalze. Eine Salzbildung an dem durch die Sulfonylgruppe substituierten Stickstoffatom der (Thio)Hamstoffgruppe führt zu Verbindungen der Formel II,

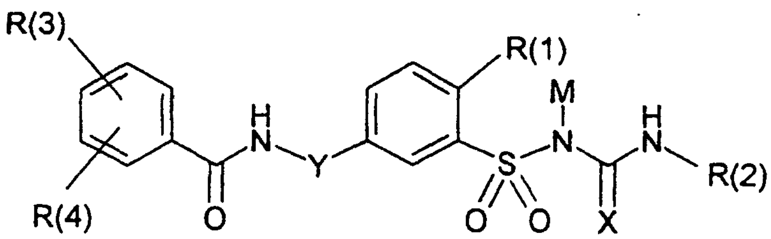

II

in der R(1 ), R(2), R(3), R(4), X und Y die oben angegebenen Bedeutungen haben und das Kation M beispielsweise für ein Alkalimetallion oder ein Äquivalent eines Erdalkalimetallions steht, zum Beispiel für das Natrium-, Kalium-, Magnesium- oder Calciumion, oder für das unsubstituierte Ammoniumion oder für ein Ammoniumion mit einem oder mehreren organischen Resten steht. Ein für M stehendes Ammoniumion kann beispielsweise auch das aus einer Aminosäure durch Protonierung erhaltene Kation sein, insbesondere das aus einer basischen Aminosäure wie zum Beispiel Lysin oder Arginin erhaltene Kation.

Verbindungen der Formel I, die eine oder mehrere basische, das heißt protonierbare, Gruppen enthalten, können in Form ihrer Säureadditionssalze mit physiologisch verträglichen anorganischen oder organischen Säuren vorliegen und erfindungsgemäß verwendet werden, zum Beispiel als Salze mit Chlorwasserstoff, Phosphorsäure, Schwefelsäure oder organischen Carbonsäuren oder Sulfonsäuren wie zum Beispiel p-Toluolsulfonsäure, Essigsäure, Weinsäure, Benzoesäure, Fumarsäure, Maleinsäure, Zitronensäure etc. Enthalten die Verbindungen der Formel I gleichzeitig saure und basische Gruppen im Molekül, so umfaßt die vorliegende Erfindung neben den geschilderten Salzformen auch innere Salze oder Betaine (Zwitterionen). Auch Säureadditionssalze können aus den Verbindungen der Formel I nach üblichen, dem Fachmann bekannten Verfahren erhalten werden, beispielsweise durch Vereinigung mit einer organischen oder anorganischen Säure in einem Lösungsmittel oder Dispergiermittel. Die vorliegende Erfindung umfaßt auch alle Salze der Verbindungen der Formel I, die sich wegen geringer physiologischer Verträglichkeit nicht direkt für eine Verwendung in Arzneimitteln eignen, aber zum Beispiel als Zwischenprodukte für chemische Reaktionen oder für die Herstellung physiologisch verträglicher Salze in Betracht kommen.

Die vorliegende Erfindung umfaßt weiterhin alle Solvate von Verbindungen der Formel I, zum Beispiel Hydrate oder Addukte mit Alkoholen, sowie Derivate der Verbindungen der Formel I wie zum Beispiel Ester oder Amide, und Prodrugs und aktive Metabolite.

In der Formel I steht Y bevorzugt für $-(CH_2)_n-$, besonders bevorzugt für $-CH_2-CH_2-$.

R(1) steht bevorzugt für

1. Phenyl, das unsubstituiert ist oder durch einen Substituenten aus der Reihe Halogen, vorzugsweise Fluor oder Chlor, $(C_1-C_4)$-Alkyl, vorzugsweise Methyl, $(C_1-C_4)$-Alkoxy, vorzugsweise Methoxy, $-S(O)_m-(C_1-C_4)$-Alkyl, vorzugs-

weise - $S(O)_m$-Methyl, Trifluormethyl und Nitro substituiert ist, wobei sich der Substituent vorzugsweise in der para-Position befindet; oder
2. monocyclisches Heteroaryl mit ein oder zwei gleichen oder verschiedenen, vorzugsweise mit einem, Ring-Heteroatomen aus der Reihe Sauerstoff, Schwefel und Stickstoff, insbesondere für Furyl, Thienyl oder Pyridyl, speziell für 2-Furyl, 2-Thienyl, 2-Pyridyl oder 3-Pyridyl; oder 3. -S-Phenyl; oder
4. $(C_2-C_3)$-Alkenyl, insbesondere Vinyl oder Allyl; oder
5. Ethinyl oder 2-Phenylethinyl, insbesondere Ethinyl.

R(2) steht bevorzugt für $(C_1-C_3)$-Alkyl, besonders bevorzugt für Methyl, Ethyl oder Isopropyl.
Bevorzugt steht einer der Reste R(3) und R(4) für Halogen, insbesondere Chlor, und der andere für $(C_1-C_4)$-Alkoxy, insbesondere $(C_1-C_3)$-Alkoxy, speziell Methoxy. Weiterhin steht bevorzugt einer der Reste R(3) und R(4) in der 2-Position und der andere in der 5-Position des Phenylringes. Besonders bevorzugt trägt der die Reste R(3) und R(4) enthaltende Benzoylrest in der 2-Position einen Rest R(3), der für $(C_1-C_4)$-Alkoxy, insbesondere $(C_1-C_3)$-Alkoxy, speziell Methoxy, steht, und in der 5-Position einen Rest R(4), der für Halogen, insbesondere Chlor, steht.
R(5) steht bevorzugt für Reste aus der Reihe Wasserstoff und Methyl, besonders bevorzugt für Wasserstoff.
n steht bevorzugt für 1, 2 oder 3, besonders bevorzugt für 2.
Bevorzugte Verbindungen der Formel I sind solche, in denen einer oder mehrere der darin enthaltenen Reste bevorzugte Bedeutungen haben, wobei alle Kombinationen von bevorzugten Substituentendefinitionen Gegenstand der vorliegenden Erfindung sind. Auch von allen bevorzugten Verbindungen der Formel I umfaßt die vorliegende Erfindung alle ihre stereoisomeren Formen und Mischungen davon in allen Verhältnissen, und ihre physiologisch verträglichen Salze.
So bilden zum Beispiel eine Gruppe von bevorzugten Verbindungen solche Verbindungen der Formel I, in der R(1) für

1. Phenyl, das unsubstituiert ist oder durch einen Substituenten aus der Reihe Halogen, $(C_1-C_4)$-Alkyl, $(C_1-C_4)$-Alkoxy, $-S(O)_m-(C_1-C_4)$-Alkyl, Trifluormethyl und Nitro substituiert ist; oder
2. monocyclisches Heteroaryl mit ein oder zwei gleichen oder verschiedenen Ring-Heteroatomen aus der Reihe Sauerstoff, Schwefel und Stickstoff; oder
3. -S-Phenyl; oder
4. $(C_2-C_3)$-Alkenyl; oder
5. Ethinyl oder 2-Phenylethinyl

steht und alle anderen Reste in der Formel I die in der obigen Definition der erfindungsgemäßen Verbindungen angegebenen Bedeutungen haben, in allen ihren stereoisomeren Formen und Mischungen davon in allen Verhältnissen, und ihre physiologisch verträglichen Salze.
Eine weitere Gruppe von bevorzugten Verbindungen bilden zum Beispiel Verbindungen der Formel Ia,

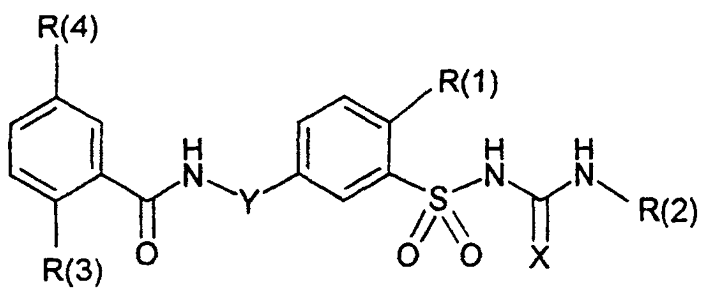

Ia

in der
Y für $-CH_2-CH_2-$ steht;
R(2) für Methyl, Ethyl oder Isopropyl steht;
R(3) für $(C_1-C_4)$-Alkoxy steht;
R(4) für Halogen steht;
und der Rest R(1) in der Formel Ia die in der obigen Definition der erfindungsgemäßen Verbindungen angegebenen Bedeutungen hat, in allen ihren stereoisomeren Formen und Mischungen davon in allen Verhältnissen, und ihre physiologisch verträglichen Salze.
Gegenstand der vorliegenden Erfindung sind auch Verfahren zur Herstellung der Verbindungen der Formel

I, die im folgenden erläutert sind und nach denen die erfindungsgemäßen Verbindungen erhältlich sind.

Verbindungen der Formel I, in der X für Schwefel steht, das heißt 2,5-substituierte Benzolsulfonylthioharnstoffe der Formel Ib,

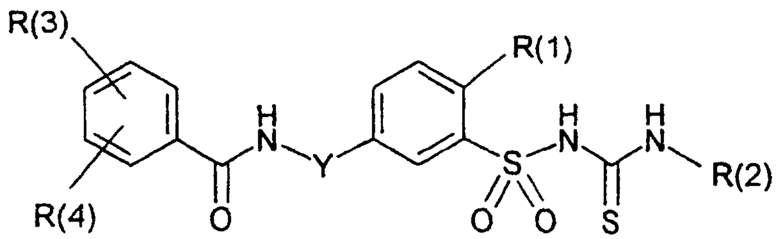

Ib

in der R(1), R(2), R(3), R(4) und Y die oben angeführten Bedeutungen haben, können zum Beispiel hergestellt werden, indem man 2,5-substituierte Benzolsulfonamide der Formel III,

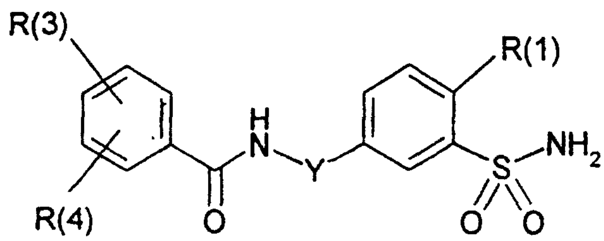

III

in der R(1), R(3), R(4) und Y die oben angeführten Bedeutungen haben, in einem inerten Lösungsmittel oder Verdünnungsmittel mit einer Base und mit einem R(2)-substituierten Isothiocyanat der Formel IV

$$R(2)\text{-}N{=}C{=}S \qquad IV$$

umsetzt, in der R(2) die oben angegebenen Bedeutungen hat. Als Basen eignen sich zum Beispiel Alkalimetall- oder Erdalkalimetallhydroxide, -hydride, -amide oder - alkoholate, wie Natriumhydroxid, Kaliumhydroxid, Calciumhydroxid, Natriumhydrid, Kaliumhydrid, Calciumhydrid, Natriumamid, Kaliumamid, Natriummethylat, Natriumethylat, Kalium-tert-butylat, oder quartäre Ammoniumhydroxide. Die Reaktion der Verbindung der Formel III mit der Base kann zunächst in einem separaten Schritt durchgeführt werden und das zunächst entstehende Salz der Formel V,

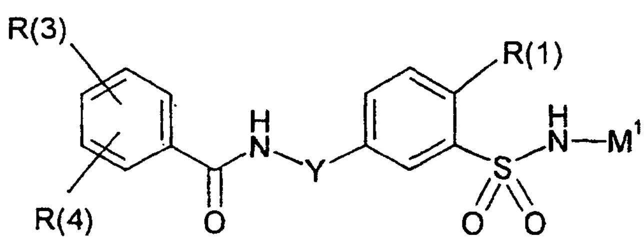

V

in der R(1 ), R(3), R(4) und Y die oben angeführten Bedeutungen haben und $M^1$ für ein Alkalimetallion, zum Beispiel Natrium oder Kalium, oder ein Äquivalent eines Erdalkalimetallions, zum Beispiel Magnesium oder Calcium, oder für ein unter den Reaktionsbedingungen inertes Ammoniumion, zum Beispiel ein quartäres Ammoniumion, steht, kann gewünschtenfalls auch zwischenisoliert werden. Das Salz der Formel V kann aber besonders vorteilhaft auch in situ

aus der Verbindung der Formel III erzeugt und direkt mit dem Isothiocyanat der Formel IV umgesetzt werden. Als inerte Lösungsmittel für die Umsetzung eignen sich zum Beispiel Ether wie Tetrahydrofuran (THF), Dioxan, Ethylenglykoldimethylether oder Diethylenglykoldimethylether, Ketone wie Aceton oder Butanon, Nitrile wie Acetonitril, Nitroverbindungen wie Nitromethan, Ester wie Ethylacetat, Amide wie Dimethylformamid (DMF) oder N-Methylpyrrolidon (NMP), Hexamethylphosphorsäuretriamid (HMPT), Sulfoxide wie Dimethylsulfoxid (DMSO) oder Kohlenwasserstoffe wie Benzol, Toluol oder Xylole. Weiterhin eignen sich auch Gemische dieser Lösungsmittel untereinander. Die Umsetzung der Verbindungen der Formel III bzw. V mit der Verbindung der Formel IV wird im allgemeinen bei Temperaturen von Raumtemperatur bis 150 °C durchgeführt.

Verbindungen der Formel I, in der X für Sauerstoff steht, das heißt 2,5-substituierte Benzolsulfonylharnstoffe der Formel Ic,

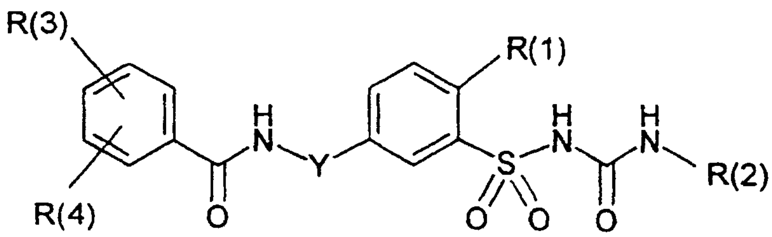

Ic

in der R(1), R(2), R(3), R(4) und Y die oben angeführten Bedeutungen haben, können zum Beispiel hergestellt werden, indem man analog der vorstehend beschriebenen Synthese der Verbindungen der Formel Ib 2,5-substituierte Benzolsulfonamide der Formel III oder deren Salze der Formel V in einem inerten Lösungsmittel oder Verdünnungsmittel mit einer Base und mit einem R(2)-substituierten Isocyanat der Formel VI

$$R(2)\text{-}N{=}C{=}O \qquad VI$$

umsetzt, in der R(2) die oben angegebenen Bedeutungen hat. Die obigen Erläuterungen zur Umsetzung der Isothiocyanate gelten für die Umsetzung der Isocyanate entsprechend.

2,5-substituierte Benzolsulfonylharnstoffe der Formel Ic lassen sich auch aus den 2,5-substituierten Benzolsulfonamiden der Formel III bzw. ihren Salzen der Formel V durch Umsetzung mit R(2)-substituierten 2,2,2-Trichloracetamiden der Formel VII,

$$Cl_3C\text{-}CO\text{-}NH\text{-}R(2) \qquad VII$$

in der R(2) die oben angegebenen Bedeutungen hat, in Gegenwart einer Base in einem inerten, hochsiedenden Lösungsmittel wie zum Beispiel DMSO herstellen. Die Verbindungen der Formel Ic können auch aus den Urethan-Derivaten der 2,5-substituierten Benzolsulfonamide der Formel III, die aus den Verbindungen der Formel III durch Umsetzung mit Chlorameisensäureestem zugänglich sind, durch Einwirkung des entsprechenden Amins der Formel R(2)-$NH_2$ in einem inerten, , hochsiedenden Lösungsmittel, zum Beispiel Toluol, bei Temperaturen bis zum Siedepunkt des jeweiligen Lösungsmittels erhalten werden (siehe beispielsweise J. Med. Chem. 38 (1995) 2357-2377 und Bioorg. Med. Chem. 5 (1997) 673-678).

2,5-substituierte Benzolsulfonylharnstoffe der Formel Ic lassen sich auch durch eine Umwandlungsreaktion (Entschwefelung) aus den entsprechenden 2,5-substituierten Benzolsulfonylthioharnstoffen der Formel Ib herstellen. Der Ersatz des Schwefelatoms in der Thioharnstoffgruppe in den Benzolsulfonylthioharnstoffen der Formel Ib durch ein Sauerstoffatom kann beispielsweise mit Hilfe von Oxiden oder Salzen von Schwermetallen oder durch Anwendung von Oxidationsmitteln wie Wasserstoffperoxid, Natriumperoxid oder salpetriger Säure durchgeführt werden.

2,5-substituierte Benzolsulfonylharnstoffe und -thioharnstoffe der Formel I lassen sich auch durch Umsetzung von Aminen der Formel R(2)-$NH_2$ mit 2,5-substituierten Benzolsulfonylisocyanaten und -isothiocyanaten der Formel VIII,

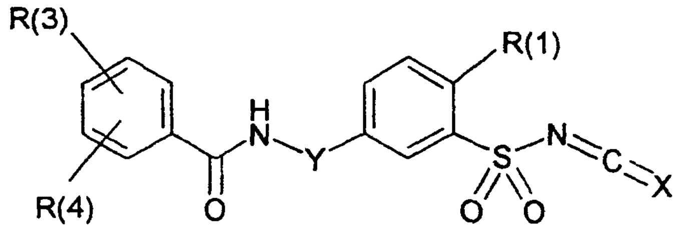

VIII

in der R(1 ), R(3), R(4), X und Y die oben angeführten Bedeutungen haben, herstellen. Die Sulfonylisocyanate der Formel VIII (X = Sauerstoff) lassen sich aus den 2,5-substituierten Benzolsulfonamiden der Formel III nach üblichen Methoden, beispielsweise mit Phosgen, erhalten. Die Herstellung der Sulfonylisothiocyanate der Formel VIII (X = Schwefel) kann durch Umsetzung der Sulfonamide der Formel III mit Alkalihydroxiden und Schwefelkohlenstoff in einem organischen Lösungsmittels wie DMF, DMSO oder NMP erfolgen. Das erhaltene Di-Alkalimetallsalz der Sulfonyldithiocarbaminsäure kann in einem inerten Lösungsmittel mit einem leichten Überschuß von Phosgen bzw. von einem Phosgenersatzstoff wie Triphosgen oder mit einem Chlorameisensäureester (2 Äquivalente) oder mit Thionylchlorid umgesetzt werden. Die so erhaltene Lösung des Sulfonylisothiocyanats kann direkt mit dem entsprechenden substituierten Amin der Formel R(2)-$NH_2$ umgesetzt werden, oder, wenn Verbindungen der Formel I hergestellt werden sollen, in der R(2) für Wasserstoff steht, mit Ammoniak umgesetzt werden.

Die 2,5-substituierten Benzolsulfonamide der Formel III als Ausgangsverbindungen für die erwähnten Syntheseverfahren der 2,5-substituierten Benzolsulfonyl(thio)hamstoffe der Formel I können nach oder analog zu bekannten Methoden hergestellt werden, wie sie in der Literatur, zum Beispiel in den Standardwerken wie Houben-Weyl, Methoden der Organischen Chemie, Georg Thieme Verlag, Stuttgart, und Organic Reactions, John Wiley & Sons, Inc., New York, sowie in den oben angegebenen Patentschriften beschrieben sind, und zwar gegebenenfalls unter geeigneter Anpassung der Reaktionsbedingungen, was dem Fachmann geläufig ist. Dabei kann man auch von an sich bekannten, hier nicht näher erwähnten Varianten Gebrauch machen. Die Ausgangsstoffe können gewünschtenfalls auch in situ gebildet werden, derart, daß man sie aus dem Reaktionsgemisch nicht isoliert, sondern sofort weiter umsetzt.

So können p-substituierte Benzolderivate der Formel IX,

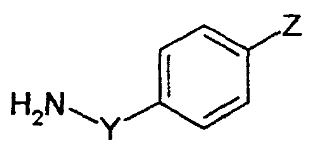

IX

in der Y die oben angeführte Bedeutung hat und Z für Brom oder Nitro steht, mit Benzoesäurederivaten zu Verbindungen der Formel X,

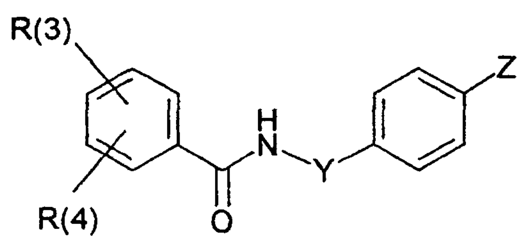

X

in der R(3), R(4) und Y die oben angegebenen Bedeutungen haben und Z für Brom oder Nitro steht, umgesetzt werden. Diese Acylierung erfolgt im allgemeinen, indem die entsprechenden Benzoesäuren zunächst in reaktive Derivate überführt werden, zum Beispiel durch Reaktion der Benzoesäure mit Carbonyl-bis-imidazol in einem inerten Lösungsmittel wie zum Beispiel THF, Dioxan oder DMF, und anschließende Umsetzung mit dem betreffenden Amin der Formel IX, gegebenenfalls in Gegenwart einer Base wie Triethylamin oder Pyridin. Als reaktive Derivate der Benzoesäuren können zum Beispiel auch Benzoesäurehalogenide oder Benzoesäureanhydride verwendet werden. Die Umsetzungen erfolgen bevorzugt bei Temperaturen von 0 °C bis zum Siedepunkt des gewählten Lösungsmittels, besonders bevorzugt bei Raumtemperatur. Alternativ kann die Acylierung des Amins der Formel IX mit den entsprechenden Benzoesäuren zum Beispiel auch in Gegenwart von Kondensationsmitteln wie zum Beispiel N,N'-Dicyclohexylcarbodiimid oder O-((Cyan-(ethoxycarbonyl)-methylen)amino)-1,1,3,3-tetramethyluronium-tetrafluoroborat (TOTU) erfolgen.

Aus den Verbindungen der Formel X, in denen Z für Nitro steht, lassen sich über eine Reduktion der Nitrogruppe mit einem Reduktionsmittel wie zum Beispiel $SnCl_2 \times 2\ H_2O$ in einem inerten Lösungsmittel wie Ethylacetat, Diazotierung der resultierenden Aminogruppe und anschließende Behandlung der intermediären Diazoverbindung mit Kaliumiodid nach an sich bekannten Verfahren, wie sie zum Beispiel in Larock, Comprehensive Organic Transformations, VCH, 1989, beschrieben sind, die entsprechenden p-Iod-substituierten Verbindungen der Formel XI erhalten,

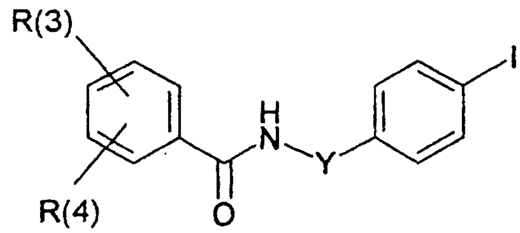

XI

in der R(3), R(4) und Y die oben angegebenen Bedeutungen haben.

Die Verbindungen der Formel XI und die Verbindungen der Formel X, in der Z für Brom steht, die zusammen als Verbindungen der Formel XII bezeichnet werden,

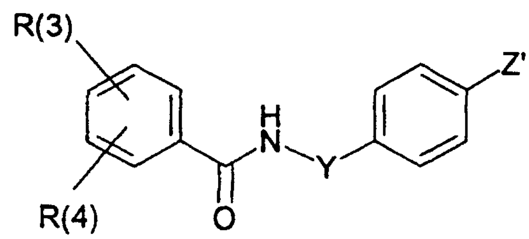

XII

in der R(3), R(4) und Y die oben angegebenen Bedeutungen haben und Z' für Brom oder Iod steht, lassen sich in bekannter Weise unter geeigneten Reaktionsbedingungen in die 2,5-substituierten Benzolsulfonamide der Formel XIII,

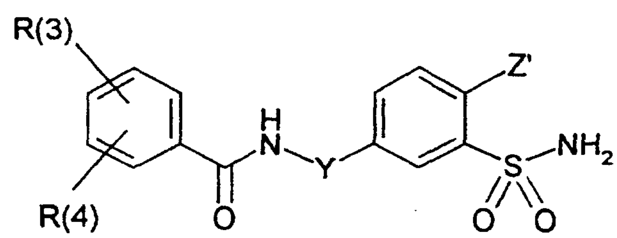

XIII

in der R(3), R(4), Y und Z' die genannten Bedeutungen haben, überführen. Die Herstellung der Sulfonamide der Formel XIII aus den Verbindungen der Formel XII kann in einem, zwei oder mehreren Schritten vollzogen werden. Insbesondere sind Verfahren bevorzugt, in denen die Acylamine der Formel XII zunächst durch elektrophile Reagenzien in Anwesenheit oder Abwesenheit von inerten Lösungsmitteln bei Temperaturen von -20 °C bis 120 °C, vorzugsweise von 0 °C bis 100 °C, in die 2,5-substituierten Benzolsulfonsäuren oder deren Derivate, wie zum Beispiel die Sulfonsäurehalogenide, überführt werden. Dafür können beispielsweise Sulfonierungen mit Schwefelsäuren oder Oleum, Halogensulfonierungen mit Halogensulfonsäuren wie Chlorsulfonsäure, Reaktionen mit Sulfurylhalogeniden in Gegenwart wasserfreier Metallhalogenide oder Reaktionen mit Thionylhalogeniden in Gegenwart von wasserfreien Metallhalogeniden mit anschließenden, in bekannter Weise durchgeführten Oxidationen zu Sulfonsäurechloriden, durchgeführt werden. Sind Sulfonsäuren die primären Reaktionsprodukte, so können diese entweder direkt oder nach Behandlung mit Aminen, wie zum Beispiel Triethylamin oder Pyridin, oder mit Alkalimetall- oder Erdalkalimetallhydroxiden oder Reagenzien, die diese basischen Verbindungen in situ bilden, in an sich bekannter Weise durch Säurehalogenide wie zum Beispiel Phosphortrihalogenide, Phosphorpentahalogenide, Thionylhalogenide oder Oxalylhalogenide, in Sulfonsäurehalogenide überführt werden. Die Überführung der Sulfonsäurederivate in die Sulfonamide der Formel XIII erfolgt in literaturbekannter Weise. Vorzugsweise werden Sulfonsäurechloride in einem inerten Lösungsmittel wie zum Beispiel Aceton bei Temperaturen von 0 °C bis 100 °C mit wäßrigem Ammoniak umgesetzt.

Die Sulfonamid-Gruppe in den Verbindungen der Formel XIII kann dann anschließend temporär durch Überführung in die N-(N,N-Dimethylaminomethylen)-sulfonamid-Gruppe geschützt werden. Die Überführung der Verbindungen der Formel XIII in die Dimethylaminomethylenverbindungen der Formel XIV,

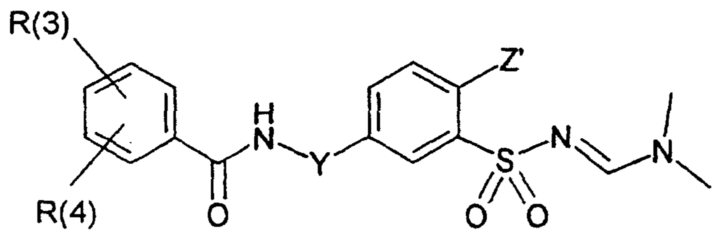

XIV

in der R(3), R(4), Y und Z' die genannten Bedeutungen haben, kann zum Beispiel durch Umsetzung der Verbindungen der Formel XIII mit N,N-Dimethylformamiddimethylacetal (J. Med. Chem. 38 (1995) 2357-2377) oder durch Umsetzung mit N,N-Dimethylformamid in Gegenwart von wasserentziehenden Mitteln wie $SOCl_2$, $POCl_3$ oder $PCl_5$ erfolgen (Liebigs Ann. (1995) 1253-1257).

Aus den Verbindungen der Formel XIV können dann die Verbindungen der Formel XV,

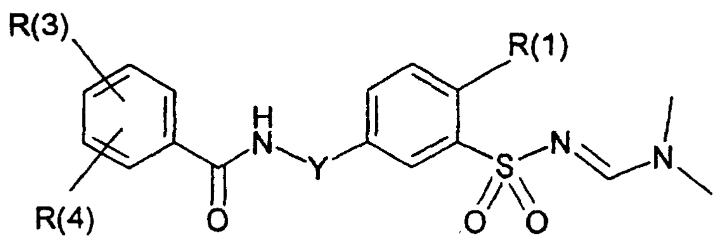

XV

in der R(1), R(3), R(4) und Y wie oben definiert sind, erhalten werden. Die Umwandlung in die Verbindungen der Formel XV kann zum Beispiel erfolgen durch Palladium-katalysierte Suzuki-Kopplung mit Arylboronsäuren wie Phenylboronsäuren oder Heteroarylboronsäuren wie Thiophenboronsäuren, oder durch Stille-Kopplung mit Trialkylstannanen, zum Beispiel mit Tributylstannylheteroaromaten wie Tributylstannyl-furan oder Trimethylstannyl-pyridin oder mit Trialkylstannyl-alkinen oder Trialkylstannyl-alkenen wie Ethinyl-tributylstannan. Die Suzuki-Kopplung erfolgt vorzugsweise mit den Arylbromiden (Verbindungen der Formel XIV mit Z' = Br) mit Palladium(II)acetat und Triphenylphosphin oder Tetrakis(triphenylphosphin)palladium als Katalysator in Gegenwart einer Base wie zum Beispiel Cäsiumcarbonat oder Kaliumcarbonat (siehe zum Beispiel Synthetic Commun. 11 (1981) 513; J. Med. Chem. 38 (1995) 2357-2377; Liebigs Ann. (1995) 1253-1257). Die Stille-Kopplung erfolgt vorzugsweise mit den Aryliodiden (Verbindungen der Formel XIV mit Z' = I) mit Bis(triphenylphosphin)palladium(11)chlorid als Katalysator (siehe zum Beispiel Tetrahedron Lett. (1986) 4407-4410). Die Herstellung geeigneter Stannane wird zum Beispiel in Tetrahedron 49 (1993) 3325-3342 beschrieben.

Verbindungen der Formel XV, in der R(1 ) für Phenylsulfanyl steht, können durch Cu(I)I-katalysierte nucleophile Substitution der Aryliodide der Formel XIV (Z' = I) mit Natrium-thiophenolat hergestellt werden (siehe zum Beispiel Chem. Lett. (1980) 1363-1364). Die so eingeführte Thioethergruppe -S- kann, wie auch eine Thioethergruppe in einer anderen Position des Moleküls der Formel I, nach Standardverfahren, zum Beispiel mit einer Persäure wie m-Chlorperbenzoesäure oder Monoperoxyphthalsäure, zur Sulfoxidgruppe -S(O)- oder zur Sulfongruppe - $S(O)_2$- oxidiert werden.

Die anschließende Abspaltung der als Sulfonamid-Schutzgruppe fungierenden Dimethylaminomethylengruppe aus den Verbindungen der Formel XV führt dann zu den gewünschten Verbindungen der Formel III. Diese Abspaltung kann sowohl unter basischen wie sauren Bedingungen durchgeführt werden. Vorzugsweise erfolgt sie durch Behandlung der Verbindungen der Formel XV in einem geeigneten Lösungsmittel, zum Beispiel Alkoholen, mit Säuren wie zum Beispiel Salzsäure.

Die Verbindungen der Formel I beeinflussen das Aktionspotential von Zellen, insbesondere von Herzmuskelzellen. Sie haben insbesondere eine normalisierende Wirkung auf ein gestörtes Aktionspotential, wie es beispielsweise bei Ischämien vorliegt, und eignen sich zum Beispiel zur Behandlung und Prophylaxe von Störungen des cardiovasculären Systems, insbesondere von Arrhythmien und deren Folgen. Die Wirksamkeit der Verbindungen der Formel I läßt sich beispielsweise in dem unten beschriebenen Modell nachweisen, in dem die Dauer des Aktionspotentials am Papillarmuskel des Meerschweinchens bestimmt wird.

Die Verbindungen der Formel I und ihre physiologisch verträglichen Salze können daher am Tier, bevorzugt am Säugetier, und insbesondere am Menschen als Arzneimittel für sich allein, in Mischungen untereinander oder in Form von pharmazeutischen Zubereitungen verwendet werden. Säugetiere, an denen die Verbindungen der Formel I angewandt oder geprüft werden können, sind zum Beispiel Affen, Hunde, Mäuse, Ratten, Kaninchen, Meerschweinchen, Katzen und größere Nutztiere wie zum Beispiel Rinder und Schweine. Gegenstand der Erfindung sind daher auch die Verbindungen der Formel I und ihre physiologisch verträglichen Salze zur Verwendung als Arzneimittel sowie pharmazeutische Präparate (oder pharmazeutische Zusammensetzungen), die eine wirksame Dosis mindestens einer Verbindung der Formel I und/oder eines physiologisch verträglichen Salzes davon als aktiven Bestandteil und einen üblichen, pharmazeutisch verträglichen Träger enthalten. Die pharmazeutischen Präparate können für eine enterale oder eine parenterale Anwendung bestimmt sein und enthalten normalerweise 0.5 bis 90 Gewichtsprozent der Verbindung der Formel I und/oder ihrer physiologisch verträglichen Salze. Die Menge an Wirkstoff der Formel I und/oder dessen physiologisch verträglichen Salzen in den pharmazeutischen Präparaten beträgt im allgemeinen ungefähr 0.2 bis ungefähr 500 mg, vorzugsweise ungefähr 1 bis ungefähr 200 mg.

Die Herstellung der erfindungsgemäßen pharmazeutischen Präparate kann in an sich bekannter Weise erfolgen. Dazu werden die Verbindungen der Formel I und/oder ihre physiologisch verträglichen Salze zusammen mit einem oder mehreren festen oder flüssigen Trägerstoffen und/oder Hilfsstoffen und, wenn gewünscht, in Kombination mit anderen Arzneimitteln, zum Beispiel Herz-Kreislauf-aktiven Arzneimitteln wie etwa Calcium-Antagonisten oder ACE-Hemmem, vermischt und in eine geeignete Dosierungsform und Darreichungsform gebracht, die dann als Arzneimittel in der Humanmedizin oder Veterinärmedizin verwendet werden kann.

Als Trägerstoffe kommen organische und anorganische Substanzen in Frage, die sich zum Beispiel für die enterale (zum Beispiel die orale oder rektale) Applikation oder für die parenterale Applikation (zum Beispiel die intravenöse, intramuskuläre oder subkutane Injektion oder Infusion) oder für topische oder perkutane Anwendungen eignen und mit den Verbindungen der Formel I nicht in unerwünschter Weise reagieren, beispielsweise Wasser, pflanzliche Öle, Wachse, Alkohole wie Ethanol, Propandiol oder Benzylalkohole, Glycerin, Polyole, Polyethylenglykole, Polypropylenglykole, Glycerintriacetat, Gelatine, Kohlenhydrate wie Lactose oder Stärke, Stearinsäure und deren Salze wie Magnesiumstearat, Talk, Lanolin und Vaseline. Zur oralen und rektalen Anwendung dienen insbesondere Arzneiformen wie Tabletten, Dragees, Kapseln, Suppositorien, Lösungen, vorzugsweise ölige oder wäßrige Lösungen, Sirupe, Säfte oder Tropfen, ferner Suspensionen oder Emulsionen. Für die topische Anwendung dienen insbesondere Salben, Cremes, Pasten, Lotionen, Gele, Sprays, Schäume, Aerosole, Lösungen oder Puder. Als Lösungsmittel für Lösungen können zum Beispiel Wasser oder Alkohole wie Ethanol, Isopropanol oder 1,2-Propandiol oder deren Gemische untereinander oder mit Wasser dienen. Als weitere Arzneiformen kommen zum Beispiel auch Implantate in Betracht. Die Verbindungen der Formel I können auch lyophilisiert und die erhaltenen Lyophilisate zum Beispiel zur Herstellung von Injektionspräparaten verwendet werden. Insbesondere für die topische Anwendung kommen auch liposomale Zubereitungen in Betracht. Die pharmazeutischen Präparate können Hilfsstoffe (oder Zusatzstoffe) wie zum Beispiel Gleit-, Konservierungs-, Dickungs-, Stabilisierungs-, Netzmittel, Mittel zur Erzielung eines Depoteffekts, Emulgatoren, Salze (zum Beispiel zur Beeinflussung des osmotischen Druckes), Puffersubstanzen, Farb-, Geschmacks- und/oder Aromastoffe enthalten. Sie können, falls erwünscht, auch einen oder mehrere weitere Wirkstoffe enthalten und/oder zum Beispiel ein oder mehrere Vitamine.

Die Verbindungen der Formel I und ihre physiologisch verträglichen Salze sind wertvolle Therapeutika, die sich nicht nur als Antiarrhythmika und zur Bekämpfung und Verhinderung der Folgen von Arrhythmien, sondern auch zur Behandlung und Prophylaxe bei anderen Störungen des cardiovasculären Systems, zum Beispiel bei Herzinsuffizienz, Ischämien oder Herztransplantationen, oder bei cerebralen Gefäßerkrankungen zum Einsatz an Menschen und Tieren eignen. Insbesondere finden sie Verwendung als Antiarrhythmika zur Behandlung von Herzrhythmusstörungen unterschiedlichster Genese, und speziell zur Verhinderung des arrhythmisch bedingten plötzlichen Herztodes. Beispiele arrhythmischer Störungen des Herzens sind supraventrikuläre Rhythmusstörungen wie etwa Vorhof-Tachycardien, Vorhof-Flattern oder paroxysomale supraventrikuläre Rhythmusstörungen, oder ventrikuläre Rhythmusstörungen wie ventrikuläre Extrasystolen, insbesondere aber lebensbedrohende ventrikuläre Tachycardien oder das besonders gefährliche Kammerflimmern. Sie eignen sich insbesondere für solche Fälle, wo Arrhythmien Folge einer Verengung eines Koronargefäßes sind, wie sie beispielsweise bei Angina pectoris oder während des akuten Herzinfakts oder als chronische Folge eines Herzinfakts auftreten. Sie sind daher insbesondere bei Postinfarktpatienten zur Verhinderung des plötzlichen Herztodes geeignet. Weitere Krankheitsbilder, bei denen derartige Rhythmusstörungen und/oder der plötzliche, arrhythmisch bedingte Herztod eine Rolle spielen, sind beispielsweise die Herzinsuffizienz oder die Herzhypertrophie als Folge eines chronisch erhöhten Blutdrucks.

Darüber hinaus sind die Verbindungen der Formel I in der Lage, eine verminderte Kontraktilität des Herzens und eine geschwächte Herzkraft positiv zu beeinflussen. Hierbei kann es sich um ein krankheitsbedingtes Nachlassen der Herzkontraktilität handeln, wie beispielsweise bei Herzinsuffizienz, aber auch um akute Fälle wie Herzversagen bei Schockeinwirkung. Ebenso kann unter dem Einfluß der Verbindungen der Formel I bei einer Herztransplantation das Herz nach erfolgter Operation seine Leistungsfähigkeit rascher und zuverlässiger wieder aufnehmen. Gleiches gilt für Operationen am Herzen, die eine vorübergehende Stillegung der Herzaktivität durch cardioplegische Lösungen erforderlich machen.

Gegenstand der vorliegenden Erfindung ist daher auch die Verwendung der Verbindungen der Formel I und/oder ihrer physiologisch verträglichen Salze zur Behandlung und Prophylaxe der genannten Krankheitsbilder, ihre Verwendung zur Herstellung von Arzneimitteln zur Verwendung bei den genannten Krankheitsbildern und Methoden zur Behandlung und Prophylaxe der genannten Krankheitsbilder.

Die Dosierung der Verbindungen der Formel I oder ihrer physiologisch verträglichen Salze hängt wie üblich von den Umständen des jeweiligen Einzelfalles ab und wird diesem vom Fachmann nach den üblichen Regeln und Vorgehensweisen angepaßt. So hängt sie von der verabreichten Verbindung der Formel I ab, von der Art und Schwere des individuellen Krankheitsbildes, vom Zustand des einzelnen Patienten oder davon, ob akut oder prophylaktisch behandelt wird. Normalerweise kommt man bei Verabreichung an einen ca. 75 kg schweren Erwachsenen mit einer Dosis aus, die mindestens ungefähr 0.01 mg, insbesondere mindestens ungefähr 0.1 mg, speziell mindestens ungefähr 1 mg beträgt, und die höchstens ungefähr 100 mg, insbesondere höchstens ungefähr 10 mg (alle Angaben jeweils in mg pro kg Körpergewicht und Tag) beträgt. Besonders geeignet ist im allgemeinen ein Dosisbereich von ungefähr 1 mg bis ungefähr 10 mg pro kg Körpergewicht und Tag. Die Dosis kann in Form einer oralen oder parenteralen Einzeldosis verabreicht werden oder in mehrere, zum Beispiel zwei, drei oder vier, Einzeldosen aufgeteilt werden. Werden akute Fälle von Herzrhythmusstörungen behandelt, beispietweise auf einer Intensivstation, kann die parenterale Verabreichung, zum Beispiel durch Injektion oder Infusion, vorteilhaft sein. Ein bevorzugter Dosisbereich in kritischen Situationen kann dann ungefähr 1 mg bis 100 mg pro kg Körpergewicht und Tag betragen und beispielsweise als intravenöse Dauerinfusion verabreicht werden. Gegebenenfalls kann es, je nach individuellem Verhalten, erforderlich werden, von den angegebenen Dosierungen nach oben oder nach unten abzuweichen.

Die Verbindungen der Formel I inhibieren ATP-sensitive Kaliumkanäle von Zellen und können außer als Arzneimittelwirkstoff in der Humanmedizin und Veterinärmedizin auch als Hilfsmittel für biochemische Untersuchungen oder als wissenschaftliches Tool eingesetzt werden, wenn eine entsprechende Beeinflussung von Ionenkanälen beabsichtigt ist, oder zur Isolierung von Kaliumkanälen. Auch können sie für diagnostische Zwecke, zum Beispiel in invitro-Diagnosen von Zellproben oder Gewebsproben, Verwendung finden. Femer können die Verbindungen der Formel I und ihre Salze als Zwischenprodukte zur Herstellung weiterer Arzneimittelwirkstoffe verwendet werden.

Die Erfindung wird durch die nachstehenden Beispiele erläutert, ohne auf diese beschränkt zu sein.

Beispiele

Abkürzungen

| | |
|---|---|
| DCI | Desorption Chemical Ionisation |
| DCM | Dichlormethan |
| DMF | N, N-Dimethylformamid |
| EE | Ethylacetat |
| ESI | Electron Spray Ionisation |
| FAB | Fast Atom Bombardment |
| Fp | Schmelzpunkt |
| h | Stunde(n) |
| Min. | Minute(n) |
| MS | Massenspektrum |
| THF | Tetrahydrofuran |

Beispiel 1

5-(2-(5-Chlor-2-methoxy-benzamido)-ethyl)-N-(methylaminothiocarbonyl)-2-phenylbenzolsulfonamid

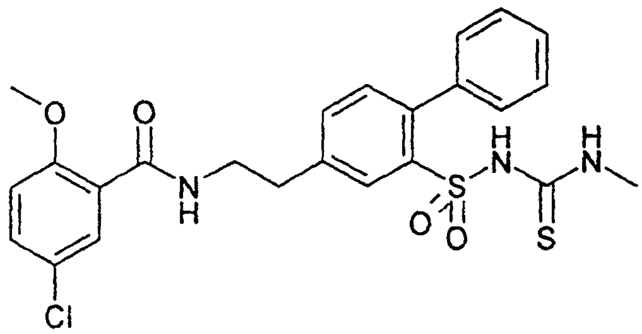

a) N-(2-(4-Bromphenyl)-ethyl)-5-chlor-2-methoxy-benzamid
Eine Lösung von 8.3 g (44.4 mmol) 5-Chlor-2-methoxy-benzoesäure und 7.8 g (48.1 mmol) Carbonyl-bis-imidazol in 200 ml absolutem THF wurde 2 h bei Raumtemperatur gerührt. Es wurden 7.7 ml (50.0 mmol) 2-(4-Bromphenyl)-ethylamin und 10 ml Triethylamin hinzugefügt und die resultierende Lösung 24 h bei Raumtemperatur gerührt. Anschließend wurde die Reaktionslösung auf Wasser gegossen, der ausgefallene Niederschlag abgesaugt und mit Wasser gewaschen. Trocknung des Niederschlages lieferte 15.8 g der Titelverbindung in Form eines schwach gelbgefärbten Feststoffes. Fp: 104 - 109 °C. $R_f$ (Kieselgel, EE/Heptan 2:1) = 0.66. MS (ESI): m/z = 368 / 370 $(M+H)^+$.

b) 2-Brom-5-(2-(5-chlor-2-methoxy-benzamido)-ethyl)-benzolsulfonamid
6.2 g (16.9 mmol) der Verbindung des Beispiels 1 a) wurden portionsweise in 55 ml Chlorsulfonsäure eingetragen und die resultierende Lösung für 6 h bei 50 °C gerührt. Die Reaktionslösung wurde dann auf 400 ml Eis getropft und der ausgefallene Niederschlag abgesaugt. Der Niederschlag wurde in 160 ml Aceton und 32 ml konz. Ammoniak verrührt, das Aceton am Rotationsverdampfer abgezogen und der pH-Wert der verbliebenen Lösung durch Zugabe von 2 N Salzsäure auf 5 eingestellt. Dann wurde mehrfach mit DCM extrahiert und die vereinigten DCM-Extrakte über Natriumsulfat getrocknet und eingeengt. Der resultierende kristalline Rückstand wurde mit Wasser und wenig Methanol gewaschen und im Hochvakuum getrocknet. Es resultierten 3.9 g der Titelverbindung in Form eines beigen Feststoffes. Fp: 190 °C. $R_f$ (Kieselgel, EE/Heptan 2:1) = 0.36. MS (DCI): m/z = 447 / 449 $(M+H)^+$.

c) 2-Brom-5-(2-(5-chlor-2-methoxy-benzamido)-ethyl)-N-(dimethylaminomethylen)-benzolsulfonamid
2.9 g (6.5 mmol) der Verbindung des Beispiels 1b) wurden in 20 ml absolutem DMF gelöst, 7.7 mmol Dimethylformamiddimethylacetal hinzugefügt und die resultierende Lösung 30 Min. bei Raumtemperatur gerührt. Es wurde zur Trockene eingeengt und der erhaltene Rückstand mit 30 ml Wasser und 30 ml 5%iger $NaHSO_4$-Lösung verrührt. Der verbliebene Rückstand wurde abgesaugt und in DCM aufgenommen. Trocknung der DCM-Lösung über Natriumsulfat und Abziehen des Lösungsmittels im Vakuum ergab ein braunes Öl, das durch Chromatographie an Kieselgel mit EE/Toluol (10:1 ) als Laufmittel gereinigt wurde. Vereinigung und Einengen der produkthaltigen Fraktionen lieferte schließlich 2.6 g der Titelverbindung als weißen Feststoff. Fp: 150 - 152 °C. $R_f$ (Kieselgel, EE/Heptan 5:1) = 0.31. MS (DCI): m/z = 502 / 504 $(M+H)^+$.

d) 5-(2-(5-Chlor-2-methoxy-benzamido)-ethyl)-N-(dimethylaminomethylen)-2-phenylbenzolsulfonamid
Zu einer Suspension von 1.0 g (2.0 mmol) der Verbindung des Beispiels 1 c) und 71 mg (0.02 mmol) Tetrakis (triphenylphosphin)palladium in 10 ml Toluol wurde eine Lösung von 244 mg (2.0 mmol) Benzolboronsäure in 10 ml Ethanol getropft. Es wurden 2.3 ml einer 2 M $Cs_2CO_3$-Lösung hinzugefügt und die Reaktionslösung für 4 h am Rückfluß (80 °C) gerührt. Anschließend wurde zur Trockene eingeengt, der resultierende Rückstand in DCM/Wasser aufgenommen und die organische Phase abgetrennt. Die abgetrennte organische Phase wurde zweimal mit Wasser gewaschen, über Natriumsulfat getrocknet und eingeengt. Reinigung des Rückstandes durch Chromatographie an Kieselgel mit EE/Toluol (8:1) als Laufmittel lieferte 684 mg der Titelverbindung in Form eines amorphen Schaumes. $R_f$ (Kieselgel, DCM/Methanol 20:1) = 0.40. MS (DCI): m/z = 500 $(M+H)^+$.

e) 5-(2-(5-Chlor-2-methoxy-benzamido)-ethyl)-2-phenyl-benzolsulfonamid
Eine Lösung von 464 mg (0.93 mmol) der Verbindung des Beispiels 1d) in 9 ml Methanol und 2.4 ml konz. Salzsäure wurde für 5 h unter Rückfluß gerührt. Anschließend wurde das Methanol im Vakuum abgezogen und der pH-Wert der verbliebenen wäßrigen Lösung durch Zugabe von 6 N Natronlauge auf 4 eingestellt. Es wurde mit EE extrahiert, die vereinigten Extrakte mit Wasser gewaschen, über Natriumsulfat getrocknet und eingeengt. Chromatographische Reinigung des resultierenden Rückstandes an Kieselgel mit EE/Toluol (10:1) als Laufmittel lieferte 325 mg der Titelverbindung als schwach gelbgefärbten Feststoff. Fp: 134 - 136 °C. $R_f$ (Kieselgel, EE/Heptan 5:1) = 0.73. MS (FAB): m/z = 445 $(M+H)^+$.

f) 5-(2-(5-Chlor-2-methoxy-benzamido)-ethyl)-N-(methylaminothiocarbonyl)-2-phenyl-benzolsulfonamid
Eine Lösung von 188 mg (0.42 mmol) der Verbindung des Beispiels 1e) und 56 mg (0.50 mmol) Kalium-tert-butylat in 2.5 ml absolutem DMF wurde 15 Min. unter einer Argon-Atmosphäre gerührt. Dann wurden 465 µl (0.46 mmol) einer 1 M Lösung von Methylisothiocyanat in DMF hinzugetropft und die resultierende Lösung für 1 h bei 80 °C gerührt. Anschließend wurde die Reaktionslösung in 30 ml 1 N Salzsäure getropft und der ausgefallene Niederschlag abgesaugt. Der Niederschlag wurde in DCM/EE gelöst, die Lösung über Natriumsulfat getrocknet und eingeengt. Der Rückstand wurde mit wenig EE verrieben und der verbliebene Niederschlag abgesaugt. Trocknung des Niederschlages im Hochvakuum lieferte 163 mg der Titelverbindung als weißen Feststoff. Fp: 192 - 196 °C. $R_f$ (Kieselgel, EE/Heptan 5:1) = 0.43. MS (DCI): m/z = 518 $(M+H)^+$.

Beispiel 2

5-(2-(5-Chlor-2-methoxy-benzamido)-ethyl)-N-(methylaminocarbonyl)-2-phenylbenzolsulfonamid

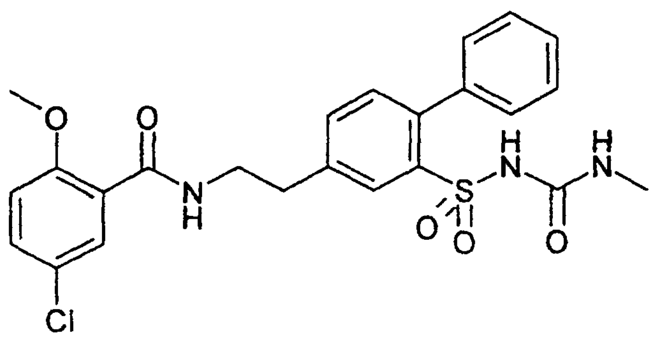

96 mg (0.19 mmol) der Verbindung des Beispiels 1f) wurden in 1 ml 1 N Natronlauge gelöst. Es wurden 80 µl 35%iger $H_2O_2$-Lösung hinzugefügt und die Reaktionslösung für 25 Min. auf dem Wasserbad erhitzt. Anschließend wurde der pH-Wert der Lösung durch Zugabe von 1 N Salzsäure auf 2 eingestellt, der ausgefallene Niederschlag abgesaugt, mit wenig Wasser gewaschen und schließlich im Hochvakuum getrocknet. Es resultierten 83 mg der Titelverbindung als weißer Feststoff. R, (Kieselgel, EE/Heptan 5:1) = 0.26. MS (DCI): m/z = 502 $(M+H)^+$.

Beispiel 3

5-(2-(5-Chlor-2-methoxy-benzamido)-ethyl)-N-(ethylaminothiocarbonyl)-2-phenylbenzolsulfonamid

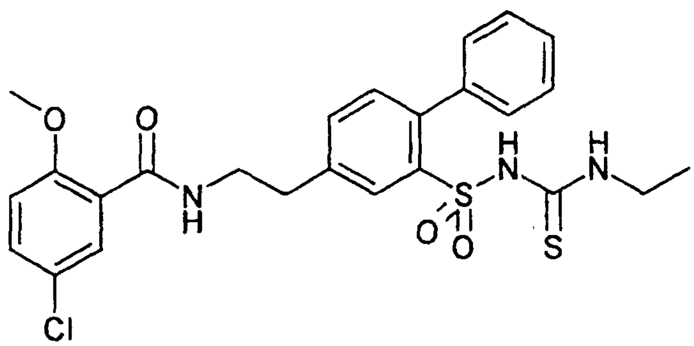

Die Herstellung erfolgte nach dem in Beispiel 1f) angeführten Verfahren, wobei Ethylisothiocyanat an Stelle von Methylisothiocyanat verwendet wurde. Aus 159 mg (0.36 mmol) der Verbindung des Beispiels 1 e) und 36 µl (0.39 mmol) Ethylisothiocyanat resultierten nach Chromatographie an Kieselgel mit EE/Methano (80:1) als Laufmittel 123 mg der Titelverbindung in Form eines weißen Feststoffs. Fp: 168 - 170 °C. $R_f$ (Kieselgel, EE/Methanol 40:1) = 0.64. MS (FAB): m/z = 532 $(M+H)^+$.

Beispiel 4

5-(2-(5-Chlor-2-methoxy-benzamido)-ethyl)-N-(ethylaminocarbonyl)-2-phenylbenzolsulfonamid

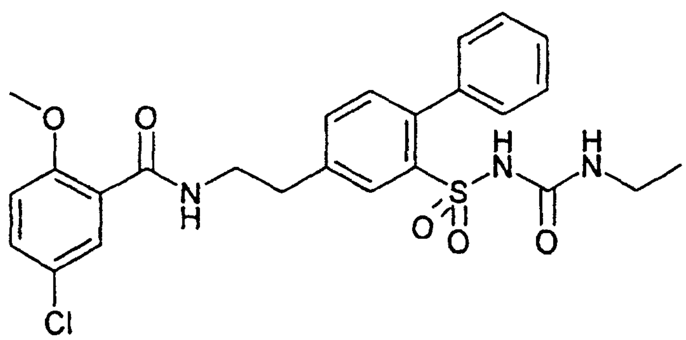

Die Herstellung erfolgte nach dem in Beispiel 2) angeführten Verfahren. Aus 70 mg (0.13 mmol) der Verbindung des Beispiels 3) resultierten 54 mg der Titelverbindung in Form eines weißen amorphen Feststoffs. R, (Kieselgel, EE/Heptan 10:1 ) = 0.41. MS (FAB): m/z = 516 $(M+H)^+$.

Beispiel 5

5-(2-(5-Chlor-2-methoxy-benzamido)-ethyl)-N-(isopropylaminothiocarbonyl)-2-phenyl-benzolsulfonamid

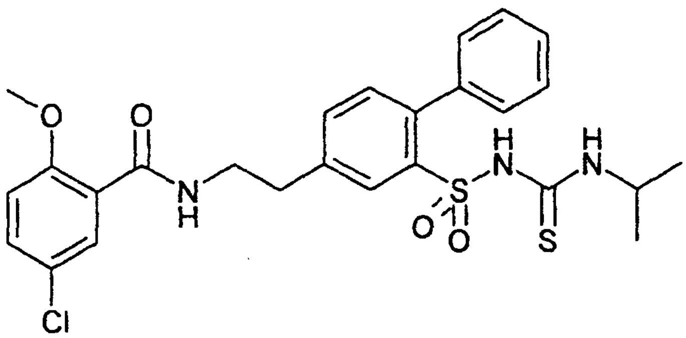

Die Herstellung erfolgte nach dem in Beispiel 1f) angeführten Verfahren, wobei Isopropylisothiocyanat an Stelle von Methylisothiocyanat verwendet wurde. Aus 162 mg (0.36 mmol) der Verbindung des Beispiels 1e) und 42 µl (0.40 mmol) Isopropylisothiocyanat resultierten nach Chromatographie an Kieselgel mit EE/Heptan (10:1) als Laufmittel 110 mg der Titelverbindung in Form eines schwach gelbgefärbten, amorphen Feststoffs. $R_f$ (Kieselgel, EE) = 0.62. MS (FAB): m/z = 546 $(M+H)^+$.

Beispiel 6

5-(2-(5-Chlor-2-methoxy-benzamido)-ethyl)-N-(isopropylaminocarbonyl)-2-phenylbenzolsulfonamid

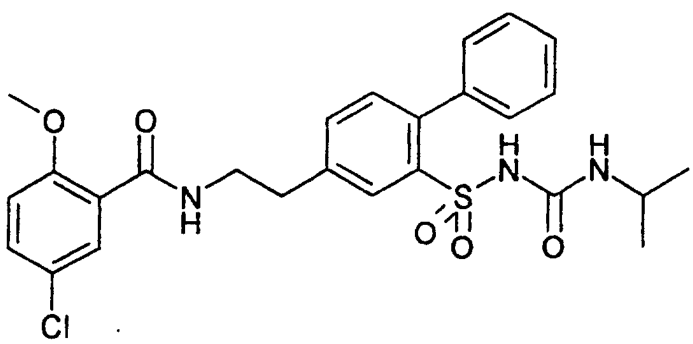

Die Herstellung erfolgte nach dem in Beispiel 2) angeführten Verfahren. Aus 70 mg (0.13 mmol) der Verbindung des Beispiels 5) resultierten 52 mg der Titelverbindung in Form eines weißen amorphen Feststoffs. $R_f$ (Kieselgel, EE) = 0.63. MS (FAB): m/z = 530 $(M+H)^+$.

Beispiel 7

5-(2-(5-Chlor-2-methoxy-benzamido)-ethyl)-2-(4-fluor-phenyl)-N-(methylaminothiocarbonyl)-benzolsulfonamid

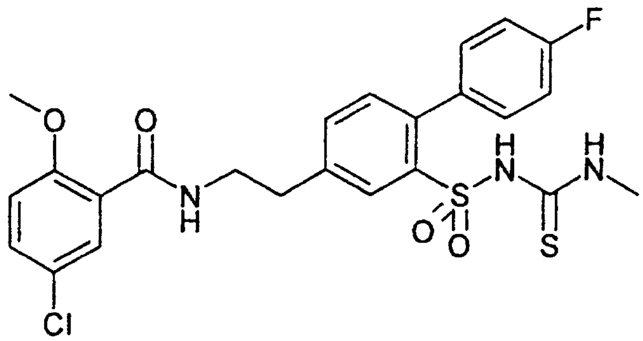

a) 5-(2-(5-Chlor-2-methoxy-benzamido)-ethyl)-N-(dimethylaminomethylen)-2-(4-fluorphenyl)-benzolsulfonamid
Die Herstellung erfolgte durch Umsetzung der Verbindung des Beispiels 1c) und 4-Fluor-benzolboronsäure nach dem in Beispiel 1d) angeführten Verfahren. Aus 1.0 g (2.0 mmol) der Verbindung des Beispiels 1c) und 280 mg (2.0 mmol) 4-Fluorbenzolboronsäure resultierten nach Chromatographie an Kieselgel mit EE/Toluol (5:1 ) als Laufmittel 229 mg der Titelverbindung als schwach gelbgefärbter, amorpher Feststoff. $R_f$ (Kieselgel, EE/Toluol 5:1) = 0.53. MS (DCI): m/z = 518 $(M+H)^+$.

b) 5-(2-(5-Chlor-2-methoxy-benzamido)-ethyl)-2-(4-fluor-phenyl)-benzolsulfonamid
Die Herstellung erfolgte aus der Verbindung des Beispiels 7a) nach dem in Beispiel 1 e) angeführten Verfahren. Es resultierten aus 222 mg (0.43 mmol) der Verbindung des Beispiels 7b) 183 mg der Titelverbindung als leicht gelbgefärbter Feststoff. Fp: 195 °C. $R_f$ (Kieselgel, EE/Heptan 10:1 ) = 0.88. MS (DCI): m/z = 463 $(M+H)^+$.

c) 5-(2-(5-Chlor-2-methoxy-benzamido)-ethyl)-2-(4-fluor-phenyl)-N-(methylaminothiocarbonyl)-benzolsulfonamid
Die Herstellung erfolgte durch Umsetzung der Verbindung des Beispiels 7b) und Methylisothiocyanat nach dem in Beispiel 1f) angeführten Verfahren. Aus 178 mg (0.39 mmol) der Verbindung des Beispiels 7b) und 423 µl (0.42 mmol) Methylisothiocyanat-Lösung wurden 160 mg der Titelverbindung als weißer Feststoff erhalten. Fp: 181 - 186 °C. $R_f$ (Kieselgel, EE/Heptan 10:1) = 0.63. MS (DCI): m/z = 537 $(M+H)^+$.

Beispiel 8

5-(2-(5-Chlor-2-methoxy-benzamido)-ethyl)-2-(4-chlor-phenyl)-N-(methylaminothiocarbonyl)-benzolsulfonamid

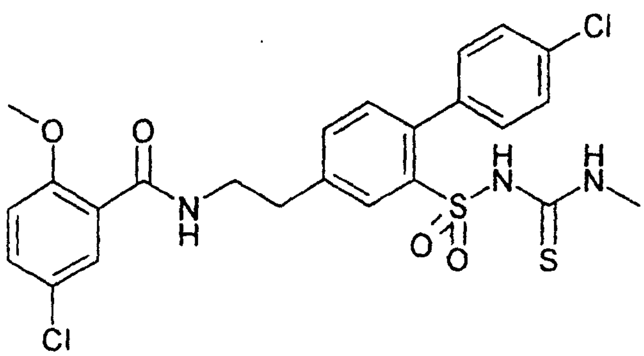

a) 5-(2-(5-Chlor-2-methoxy-benzamido)-ethyl)-2-(4-chlor-phenyl)-N-(dimethylaminomethylen}-benzolsulfonamid
Die Herstellung erfolgte durch Umsetzung der Verbindung des Beispiels 1c) und 4-Chlor-benzolboronsäure nach dem in Beispiel 1 d) angeführten Verfahren. Aus 1.0 g (2.0 mmol) der Verbindung des Beispiels 1 c) und 311 mg (2.0 mmol) 4-Chlorbenzolboronsäure resultierten nach Chromatographie an Kieselgel mit EE/Toluol (5:1) als Laufmittel 382 mg der Titelverbindung als schwach gelbgefärbter, fester Schaum. $R_f$ (Kieselgel, EE/Toluol 5:1) = 0.58. MS (DCI): m/z = 534 $(M+H)^+$.

b) 5-(2-(5-Chlor-2-methoxy-benzamido)-ethyl)-2-(4-chlor-phenyl)-benzolsulfonamid Die Herstellung erfolgte aus der Verbindung des Beispiels 8a) nach dem in Beispiel 1e) angeführten Verfahren. Es resultierten aus 376 mg (0.71 mmol) der Verbindung des Beispiels 8a) 295 mg der Titelverbindung als leicht gelbgefärbter Feststoff. Fp: 185 °C. $R_f$ (Kieselgel, EE/Heptan 5:1) = 0.72. MS (DCI): m/z = 479 $(M+H)^+$.

c) 5-(2-(5-Chlor-2-methoxy-benzamido)-ethyl)-2-(4-chlor-phenyl)-N-(methylaminothiocarbonyl)-benzolsulfonamid
Die Herstellung erfolgte durch Umsetzung der Verbindung des Beispiels 8b) und Methylisothiocyanat nach dem in Beispiel 1f) angeführten Verfahren. Aus 180 mg (0.38 mmol) der Verbindung des Beispiels 8b) und 413 µl (0.41 mmol) Methylisothiocyanat-Lösung wurden 150 mg der Titelverbindung als weißer Feststoff erhalten. Fp: 189 - 192 °C. $R_f$ (Kieselgel, EE/Heptan 5:1) = 0.53. MS (FAB): m/z = 552 $(M+H)^+$.

Beispiel 9

5-(2-(5-Chlor-2-methoxy-benzamido)-ethyl)-2-(4-methoxy-phenyl)-N-(methylaminothiocarbonyl)-benzolsulfonamid

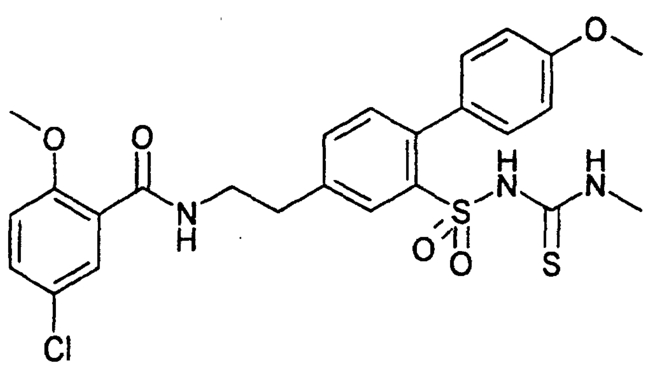

a) 5-(2-(5-Chlor-2-methoxy-benzamido)-ethyl)-N-(dimethylaminomethylen)-2-(4-methoxy-phenyl)-benzolsulfonamid
Die Herstellung erfolgte durch Umsetzung der Verbindung des Beispiels 1 c) und 4-Methoxy-benzolboronsäure nach dem in Beispiel 1 d) angeführten Verfahren. Aus 638 mg (1.27 mmol) der Verbindung des Beispiels 1c) und 193 mg (1.27 mmol) 4-Methoxy-benzolboronsäure resultierten nach Chromatographie an Kieselgel mit EE/Toluol (20:1 ) als Laufmittel 249 mg der Titelverbindung als schwach gelbgefärbter, amorpher Feststoff. $R_f$ (Kieselgel, EE/Toluol 8:1) = 0.38. MS (DCI): m/z = 530 $(M+H)^+$.

b) 5-(2-(5-Chlor-2-methoxy-benzamido)-ethyl)-2-(4-methoxy-phenyl)-benzolsulfonamid
Die Herstellung erfolgte aus der Verbindung des Beispiels 9a) nach dem in Beispiel 1 e) angeführten Verfahren. Es resultierten aus 249 mg (0.47 mmol) der Verbindung des Beispiels 9a) 165 mg der Titelverbindung als beiger Feststoff. Fp: 205 - 208 °C. $R_f$ (Kieselgel, EE/Heptan 8:1 ) = 0.70. MS (DCI): m/z = 475 $(M+H)^+$.

c) 5-(2-(5-Chlor-2-methoxy-benzamido)-ethyl)-2-(4-methoxy-phenyl)-N-(methylaminothiocarbonyl)-benzolsulfonamid
Die Herstellung erfolgte durch Umsetzung der Verbindung des Beispiels 9b) und Methylisothiocyanat nach dem in Beispiel 1f) angeführten Verfahren. Aus 159 mg (0.34 mmol) der Verbindung des Beispiels 9b) und 368 µl (0.37 mmol) Methylisothiocyanat-Lösung wurden 142 mg der Titelverbindung als weißer Feststoff erhalten. Fp: 185 - 188 °C. $R_f$ (Kieselgel, EE/Heptan 5:1) = 0.36. MS (FAB): m/z = 548 $(M+H)^+$.

Beispiel 10

5-(2-(5-Chlor-2-methoxy-benzamido)-ethyl)-N-(ethylaminothiocarbonyl)-2-(4-methylthio-phenyl)-benzolsulfonamid

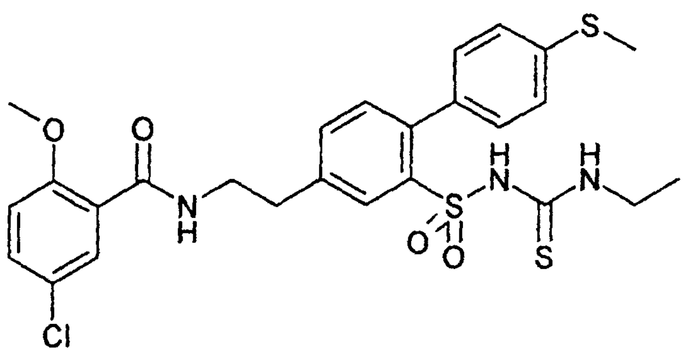

a) 5-(2-(5-Chlor-2-methoxy-benzamido)-ethyl)-N-(dimethylaminomethylen)-2-(4-methylthio-phenyl)-benzolsulfonamid

Die Herstellung erfolgte durch Umsetzung der Verbindung des Beispiels 1 c) und 4-Methylthio-benzolboronsäure nach dem in Beispiel 1 d) angeführten Verfahren. Aus 5.0 g (9.95 mmol) der Verbindung des Beispiels 1c) und 1.67 g (9.95 mmol) 4-Methylthio-benzolboronsäure resultierten nach Chromatographie an Kieselgel mit EE/Toluol (5:1) als Laufmittel 3.17 g der Titelverbindung als weißer, amorpher Schaum. $R_f$ (Kieselgel, EE/Toluol 5:1) = 0.42. MS (DCI): m/z = 546 $(M+H)^+$.

b) 5-(2-(5-Chlor-2-methoxy-benzamido)-ethyl)-2-(4-methylthio-phenyl)-benzolsulfonamid

Die Herstellung erfolgte aus der Verbindung des Beispiels 10a) nach dem in Beispiel 1e) angeführten Verfahren. Es resultierten aus 1.0 g (1.83 mmol) der Verbindung des Beispiels 10a) 634 mg der Titelverbindung als beiger Feststoff. Fp: 165 - 168 °C. $R_f$ (Kieselgel, EE/Heptan 5:1) = 0.69. MS (DCI): m/z = 491 $(M+H)^+$.

c) 5-(2-(5-Chlor-2-methoxy-benzamido)-ethyl)-N-(ethylaminothiocarbonyl)-2-(4-methylthio-phenyl)-benzolsulfonamid

Die Herstellung erfolgte durch Umsetzung der Verbindung des Beispiels 10b) und Ethylisothiocyanat nach dem in Beispiel 1f) angeführten Verfahren. Aus 225 mg (0.46 mmol) der Verbindung des Beispiels 10b) und 46 µl (0.50 mmol) Ethylisothiocyanat wurden 205 mg der Titelverbindung als weißer Feststoff erhalten. Fp: 178 - 179 °C. $R_f$ (Kieselgel, EE/Heptan 5:1 ) = 0.62. MS (DCI): m/z = 578 $(M+H)^+$.

Beispiel 11

5-(2-(5-Chlor-2-methoxy-benzamido)-ethyl)-N-(methylaminothiocarbonyl)-2-(4-methylsulfonyl-phenyl)-benzolsulfonamid

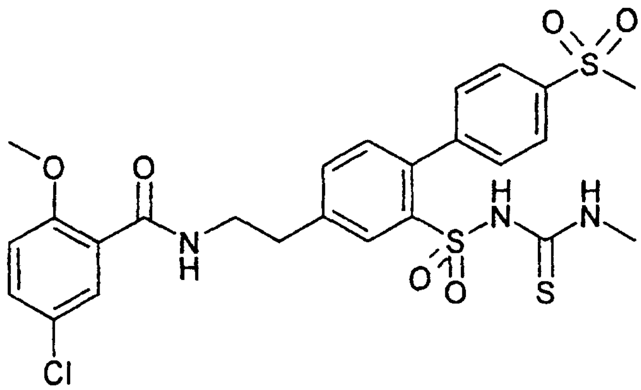

a) 5-(2-(5-Chlor-2-methoxy-benzamido)-ethyl)-N-(dimethylaminomethylen)-2-(4-methylsulfonyl-phenyl)-benzolsulfonamid
Eine Lösung von 1.15 g (2.10 mmol) der Verbindung des Beispiels 10a) und 1.0 g (4.21 mmol) 3-Chlorperoxybenzoesäure in 60 ml DCM wurde für 2 h bei Raumtemperatur gerührt. Die Reaktionslösung wurde anschließend mit 115 ml 10%iger Natriumbisulfit-Lösung versetzt, die organische Phase abgetrennt und die wäßrige Phase mit EE extrahiert. Die vereinigten organischen Phasen wurden über Natriumsulfat getrocknet, eingeengt und der resultierende Rückstand durch Chromatographie an Kieselgel mit EE/Toluol (5:1) gereinigt. Es wurden 1.35 g der Titelverbindung als weißer, amorpher Schaum erhalten. $R_f$ (Kieselgel, EE) = 0.40. MS (DCI): m/z = 578 $(M+H)^+$.

b) 5-(2-(5-Chlor-2-methoxy-benzamido)-ethyl)-2-(4-methylsulfonyl-phenyl)-benzolsulfonamid
Die Herstellung erfolgte aus der Verbindung des Beispiels 11 a) nach dem in Beispiel 1e) angeführten Verfahren. Aus 1.35 g (2.34 mmol) der Verbindung des Beispiels 11a) resultierten 1.11 g der Titelverbindung in Form eines weißen Feststoffs. Fp: 114 °C. $R_f$ (Kieselgel, EE) = 0.62. MS (DCI): m/z = 523 $(M+H)^+$.

c) 5-(2-(5-Chlor-2-methoxy-benzamido)-ethyl)-N-(methylaminothiocarbonyl)-2-(4-methylsulfonyl-phenyl)-benzolsulfonamid
Die Herstellung erfolgte aus der Verbindung des Beispiels 11 b) nach dem in Beispiel 1f) angeführten Verfahren. Aus 150 mg (0.29 mmol) der Verbindung des Beispiels 11b) resultierten nach chromatographischer Reinigung an Kieselgel mit EE als Laufmittel 107 mg der Titelverbindung als weißer, amorpher Feststoff. $R_f$ (Kieselgel, EE) = 0.19. MS (FAB): m/z = 596 $(M+H)^+$.

Beispiel 12

5-(2-(5-Chlor-2-methoxy-benzamido)-ethyl)-N-(methylaminothiocarbonyl)-2-(2-thienyl)-benzolsulfonamid

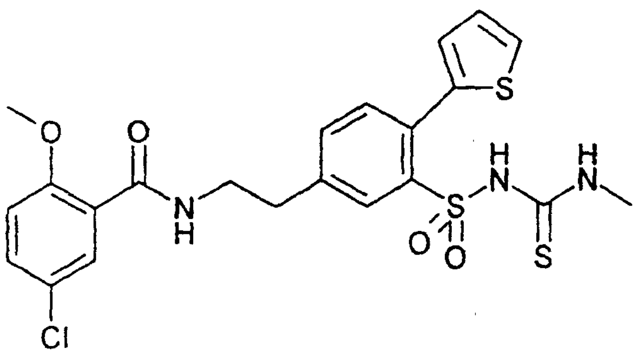

a) 5-(2-(5-Chlor-2-methoxy-benzamido)-ethyl)-N-(dimethylaminomethylen)-2-(2-thienyl)-benzolsulfonamid
Die Herstellung erfolgte durch Umsetzung der Verbindung des Beispiels 1 c) mit 2-Thiophenboronsäure nach dem in Beispiel 1 d) angeführten Verfahren. Aus 3.0 g (5.97 mmol) der Verbindung des Beispiels 1 c) und 766 mg (5.97 mmol) 2-Thiophenboronsäure resultierten nach chromatographischer Reinigung an Kieselgel mit EE/Toluol (8:1 ) als Laufmittel 580 mg der Titelverbindung als amorpher Feststoff. $R_f$ (Kieselgel, EE/Toluol 8:1 ) = 0.37. MS (FAB): m/z = 506 $(M+H)^+$.

b) 5-(2-(5-Chlor-2-methoxy-benzamido)-ethyl)-2-(2-thienyl)-benzolsulfonamid
Die Herstellung erfolgte aus der Verbindung des Beispiels 12a) nach dem in Beispiel 1 e) angeführten Verfahren. Aus 500 mg (0.99 mmol) der Verbindung des Beispiels 12a) resultierten 282 mg der Titelverbindung als blaßgelber, amorpher Schaum. $R_f$ (Kieselgel, EE/Toluol 8:1) = 0.78. MS (FAB): m/z = 451 $(M+H)^+$.

c) 5-(2-(5-Chlor-2-methoxy-benzamido)-ethyl)-N-(methylaminothiocarbonyl)-2-(2-thienyl)-benzolsulfonamid
Die Herstellung erfolgte aus der Verbindung des Beispiels 12b) nach dem in Beispiel 1f) angeführten Verfahren. Aus 161 mg (0.36 mmol) der Verbindung des Beispiels 12b) resultierten nach chromatographischer Reinigung an Kieselgel mit EE/Methanol (40:1 ) als Laufmittel 141 mg der Titelverbindung als blaßgelber Feststoff. Fp: 189 - 191 °C. $R_f$ (Kieselgel, EE/Heptan 10:1 ) = 0.27. MS (FAB): m/z = 524 $(M+H)^+$.

Beispiel 13

5-(2-(5-Chlor-2-methoxy-benzamido)-ethyl)-N-(methylaminocarbonyl)-2-(2-thienyl)-benzolsulfonamid

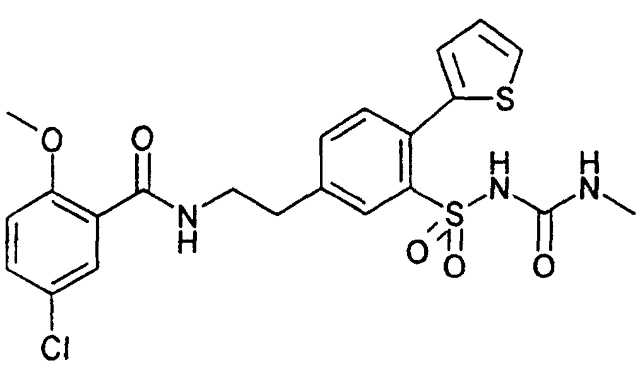

Die Herstellung erfolgte aus der Verbindung des Beispiels 12c) nach dem in Beispiel 2) angeführten Verfahren. Aus 70 mg (0.13 mmol) der Verbindung des Beispiels 12c) wurden 61 mg der Titelverbindung als weißer, amorpher Feststoff erhalten. R, (Kieselgel, EE/Heptan 10:1 ) = 0.23. MS (DCI): m/z = 508 $(M+H)^+$.

Beispiel 14

5-(2-(5-Chlor-2-methoxy-benzamido)-ethyl)-2-(2-furyl)-N-(methylaminothiocarbonyl)-benzolsulfonamid

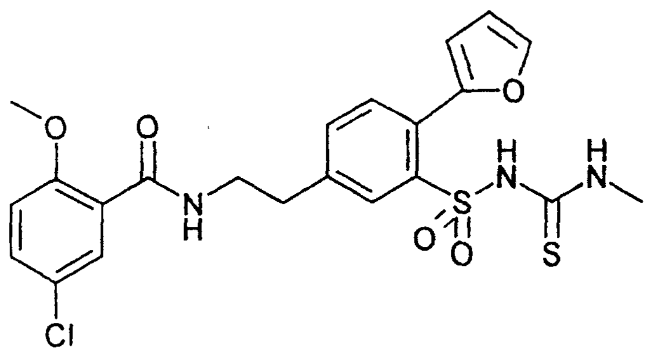

a) 5-Chlor-2-methoxy-N-(2-(4-nitrophenyl)-ethyl)-benzamid
Eine Lösung von 8.8 g (47.2 mmol) 5-Chlor-2-methoxy-benzoesäure und 8.5 g (51.9 mmol) Carbonyl-bis-imidazol in 180 ml absolutem THF wurde für 2 h bei Raumtemperatur gerührt. Es wurden 10 g (49.7 mmol) 2-(4-Nitrophenyl)-ethylamin-Hydrochlorid und 9.4 ml Triethylamin hinzugegeben und die resultierende Lösung anschließend über Nacht bei Raumtemperatur gerührt. Die Reaktionslösung wurde dann langsam auf 1.2 l 1 N Salzsäure gegossen, der ausgefallene Niederschlag abfiltriert und mit Wasser gewaschen. Trocknung des Niederschlages im Hochvakuum lieferte 13.8 g der Titelverbindung als weißen Feststoff. Fp: 159 - 160 °C. $R_f$ (Kieselgel, EE/Heptan 4:1) = 0.46. MS (ESI): m/z = 334 $(M+H)^+$.

b) N-(2-(4-Aminophenyl)-ethyl)-5-chlor-2-methoxy-benzamid
Eine Suspension aus 13.7 g (46.1 mmol) der Verbindung des Beispiels 14a) und 60 g (0.27 mol) $SnCl_2$ x 2 $H_2O$ in 400 ml EE wurde unter Rühren 2 h am Rückfluß erhitzt. Die Reaktionsmischung wurde nach dem Abkühlen mit 400 ml einer 10%igen $NaHCO_3$-Lösung versetzt und der ausgefallene Niederschlag abgesaugt. Der Niederschlag wurde in EE aufgenommen und die wäßrige Phase mit EE extrahiert. Die vereinigten EE-Lösungen wurden über Natriumsulfat getrocknet und eingeengt. Trocknung des verbliebenen Rückstands im Hochvakuum lieferte 12.1 g der Titelverbindung. $R_f$ (Kieselgel, EE/Heptan 4:1) = 0.23. MS (DCI): m/z = 305 $(M+H)^+$.

c) 5-Chlor-N-(2-(4-iod-phenyl)-ethyl)-2-methoxy-benzamid
9.0 g (29.6 mmol) der Verbindung des Beispiels 14b) wurden in einer Lösung aus 30 ml Wasser und 7.5 ml konz. Salzsäure suspendiert. Nach Kühlen der Suspension auf 0 °C wurde eine Lösung von 2.1 g (30.6 mmol) Natriumnitrit in 6 ml Wasser zugetropft. Nach 5 Min. Rühren bei 0 °C wurde dann eine Lösung von 5.0 g (30.6 mmol) Kaliumiodid in 7.5 ml Wasser zugetropft und die resultierende Lösung unter Zugabe von 40 ml Wasser 1 h bei Raumtemperatur und dann 10 Min. bei 40 °C gerührt. Nach dem Abkühlen und nach Zugabe von $NaHSO_3$ wurde die Lösung mehrfach mit DCM extrahiert, die vereinigten organischen Phasen mit Wasser gewaschen und über Natriumsulfat getrocknet. Das Lösungsmittel wurde im Vakuum entfernt und der Rückstand an Kieselgel chromatographisch mit EE/Heptan (1:2) als Laufmittel gereinigt. Es wurden 5.9 g der Titelverbindung erhalten. $R_f$ (Kieselgel, EE/Heptan 2:1) = 0.48. MS (DCI): m/z = 415 $(M+H)^+$.

d) 5-(2-(5-Chlor-2-methoxy-benzamido)-ethyl)-2-iod-benzolsulfonamid
Die Substanz wurde analog Beispiel 1b) unter Verwendung von 5.6 g (13.5 mmol) der Verbindung des Beispiels 14c) hergestellt. Es wurden 3.3 g der Titelverbindung erhalten. $R_f$ (Kieselgel, EE/Heptan 4:1 ) = 0.35. MS (DCI): m/z = 494 $(M+H)^+$.

e) 5-(2-(5-Chlor-2-methoxy-benzamido)-ethyl)-N-(dimethylaminomethylen)-2-iodbenzolsulfonamid

Die Herstellung erfolgte aus der Verbindung des Beispiels 14d) nach dem in Beispiel 1 c) angeführten Verfahren. Aus 2.2 g (4.4 mmol) der Verbindung des Beispiels 14d) wurden nach Reinigung durch Chromatographie an Kieselgel mit EE/Heptan (4:1 ) als Laufmittel 1.7 g der Titelverbindung als weißer Feststoff erhalten. Fp: 183 - 186 °C. $R_f$ (Kieselgel, EE/Heptan 4:1 ) = 0.14. MS (DCI): m/z = 549 $(M+H)^+$.

f) 5-(2-(5-Chlor-2-methoxy-benzamido)-ethyl)-N-(dimethylaminomethylen)-2-(2-furyl)-benzolsulfonamid
0.5 g (0.91 mmol) der Verbindung des Beispiels 14e) wurden in 5 ml absolutem THF suspendiert. Es wurden nacheinander 40 mg (0.005 mmol) Bis(triphenylphosphin)palladium(II)chlorid und 450 mg (1.26 mmol) 2-(Tributylstannyl)-furan hinzugefügt und die resultierende Reaktionsmischung 20 h unter Rückfluß gerührt. Nach Zusatz von 10 ml Diethylether wurde die Reaktionslösung über neutrales Aluminiumoxid filtriert, das anschließend mehrfach mit Diethylether und EE gewaschen wurde. Die vereinigten Filtrate wurden zweimal mit Wasser, getrocknet und eingeengt. Die Reinigung des resultierenden Rückstandes durch Chromatographie an Kieselgel mit EE/Heptan (4:1 ) als Laufmittel lieferte 415 mg der Titelverbindung. $R_f$ (Kieselgel, EE/Heptan 4:1) = 0.15. MS (FAB): m/z = 490 $(M+H)^+$.

g) 5-(2-(5-Chlor-2-methoxy-benzamido)-ethyl)-2-(2-furyl)-benzolsulfonamid
Die Herstellung erfolgte aus der Verbindung des Beispiels 14f) nach dem in Beispiel 1 e) angeführten Verfahren. Aus 475 mg (0.91 mmol) der Verbindung des Beispiels 14f) resultierten 280 mg der Titelverbindung als beiger Feststoff. Fp: 195 °C. $R_f$ (Kieselgel, EE/Heptan 4:1 ) = 0.42. MS (FAB): m/z = 435 $(M+H)^+$.

h) 5-(2-(5-Chlor-2-methoxy-benzamido)-ethyl)-2-(2-furyl)-N-(methylaminothiocarbonyl)-benzolsulfonamid
Die Herstellung erfolgte aus der Verbindung des Beispiels 14g) nach dem in Beispiel 1f) angeführten Verfahren. Aus 150 mg (0.35 mmol) der Verbindung des Beispiels 14g) resultierten nach chromatographischer Reinigung an Kieselgel mit DCM/EE (4:1) als Laufmittel 150 mg der Titelverbindung als blaßgelber Feststoff. Fp: 145 - 146 °C. $R_f$ (Kieselgel, EE/Heptan 4:1) = 0.25. MS (DCI): m/z = 508 $(M+H)^+$.

Beispiel 15

5-(2-(5-Chlor-2-methoxy-benzamido)-ethyl)-2-(2-furyl)-N-(methylaminocarbonyl)-benzolsulfonamid

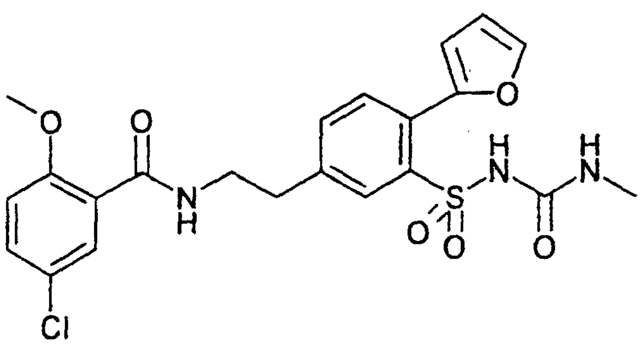

Die Herstellung erfolgte aus der Verbindung des Beispiels 14h) nach dem in Beispiel 2) angeführten Verfahren. Aus 80 mg (0.16 mmol) der Verbindung des Beispiels 14h) resultierten 60 mg der Titelverbindung in Form eines weißen Feststoffs. Fp: 118 - 120 °C. $R_f$ (Kieselgel, EE/Heptan 4:1) = 0.13. MS (DCI): m/z = 492 $(M+H)^+$.

Beispiel 16

5-(2-(5-Chlor-2-methoxy-benzamido)-ethyl)-N-(methylaminothiocarbonyl)-2-(2-pyridyl)-benzolsulfonamid

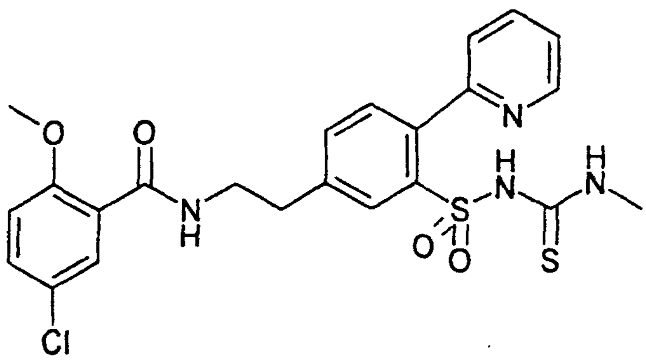

a) 2-(Trimethylstannyl)-pyridin
Unter einer Argon-Atmosphäre wurden 7.5 ml n-Butyllithium (15%ig in Hexan, 12 mmol) zu einer auf -78 °C gekühlten Lösung von 1.9 g (12.0 mmol) 2-Brompyridin in 50 ml absolutem THF getropft. Nach 1 h Rühren bei -78 °C wurden 12 ml (12 mmol) einer 1 M Lösung von Trimethylzinnchlorid in THF zugetropft und eine weitere Stunde bei -78 °C gerührt. Die Reaktionslösung wurde auf 0 °C erwärmt und mit Wasser versetzt. Die organische Phase wurde abgetrennt und die wäßrige Phase mehrfach mit Diethylether extrahiert. Die vereinigten organischen Phasen wurden über Natriumsulfat getrocknet und eingeengt. Der Rückstand wurde im Vakuum (0.3 bar) im Kugelrohr destilliert. Es resultierten 1.9 g der Titelverbindung als farbloses Öl.

b) 5-(2-(5-Chlor-2-methoxy-benzamido)-ethyl)-N-(dimethylaminomethylen)-2-(2-pyridyl)-benzolsulfonamid
400 mg (0.73 mmol) der Verbindung des Beispiels 14e) wurden in 5 ml absolutem THF suspendiert. Es wurden nacheinander 32 mg (0.004 mmol) Bis(triphenylphosphin)palladium(II)chlorid, 10 mg Lithiumchlorid und 199 mg (0.82 mmol) der Verbindung des Beispiels 16a) hinzugefügt und die resultierende Reaktionsmischung 7 h unter Rückfluß gerührt. Es wurden 10 mg Lithiumchlorid und 10 mg Kupfer(I)iodid hinzugefügt und das Gemisch weitere 2 h zum Rückfluß erhitzt. Die Reaktionslösung wurde nach dem Abkühlen mit 10 ml EE versetzt, filtriert und das Filtrat eingeengt. Die Reinigung des resultierenden Rückstandes durch Chromatographie an Kieselgel mit DCM/EE (4:1 ) als Laufmittel lieferte 265 mg der Titelverbindung. $R_f$ (Kieselgel, DCM/EE 4:1 ) = 0.05. MS (DCI): m/z = 501 $(M+H)^+$.

c) 5-(2-(5-Chlor-2-methoxy-benzamido)-ethyl)-2-(2-pyridyl)-benzolsulfonamid
Die Herstellung erfolgte aus der Verbindung des Beispiels 16b) nach dem in Beispiel 1e) angeführten Verfahren. Aus 250 mg (0.50 mmol) der Verbindung des Beispiels 16b) resultierten 160 mg der Titelverbindung als beiger Feststoff. Fp: 202 - 203 °C. $R_f$ (Kieselgel, DCM/EE 4:1 ) = 0.29. MS (DCI): m/z = 446 $(M+H)^+$.

d) 5-(2-(5-Chlor-2-methoxy-benzamido)-ethyl)-N-(methylaminothiocarbonyl)-2-(2-pyridyl)-benzolsulfonamid
Die Herstellung erfolgte aus der Verbindung des Beispiels 16c) nach dem in Beispiel 1f) angeführten Verfahren. Aus 150 mg (0.34 mmol) der Verbindung des Beispiels 16c) resultierten nach chromatographischer Reinigung an Kieselgel mit DCM/EE (4:1 ) als Laufmittel 118 mg der Titelverbindung als blaßgelber Feststoff. Fp: 85 - 86 °C. $R_f$ (Kieselgel, EE/Heptan 4:1 ) = 0.21. MS (FAB): m/z = 519 $(M+H)^+$.

Beispiel 17

5-(2-(5-Chlor-2-methoxy-benzamido)-ethyl)-N-(methylaminocarbonyl)-2-(2-pyridyl)-benzolsulfonamid

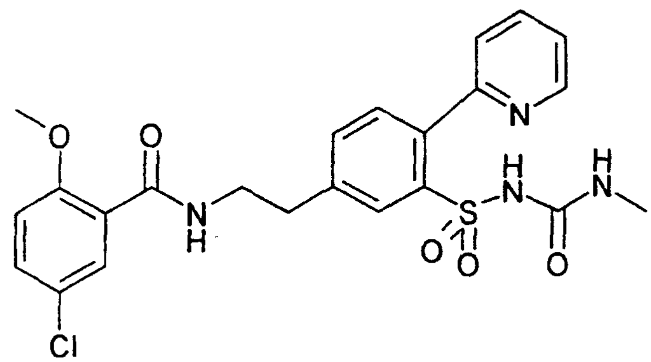

Die Herstellung erfolgte aus der Verbindung des Beispiels 16d) nach dem in Beispiel 2) angeführten Verfahren. Aus 75 mg (0.14 mmol) der Verbindung des Beispiels 16d) resultierten 55 mg der Titelverbindung in Form eines weißen Feststoffs. Fp: 160 °C. $R_f$ (Kieselgel, DCM/EE 4:1) = 0.22. MS (FAB): m/z = 503 $(M+H)^+$.

Beispiel 18

2-Allyl-5-(2-(5-chlor-2-methoxy-benzamido)-ethyl)-N-(methylaminocarbonyl)-benzolsulfonamid

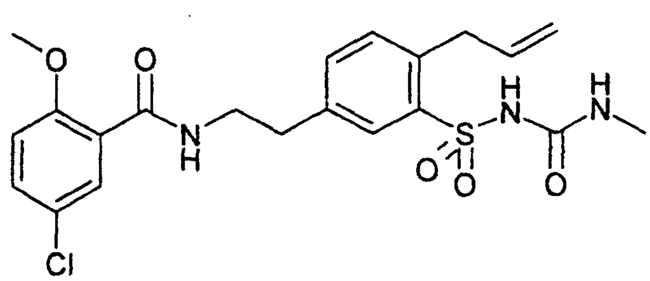

a) 2-Allyl-5-(2-(5-chlor-2-methoxy-benzamido)-ethyl)-N-(dimethylaminomethylen)-benzolsulfonamid
Die Herstellung erfolgte durch Umsetzung der Verbindung des Beispiels 14e) mit Allyl-tributylzinn nach dem in Beispiel 14f) angeführten Verfahren. Aus 800 mg (1.46 mmol) der Verbindung des Beispiels 14e) und 510 mg (1.65 mmol) Allyl-tributylzinn wurden nach chromatographischer Reinigung an Kieselgel mit EE/Heptan (4:1 ) als Laufmittel 600 mg der Titelverbindung erhalten. $R_f$ (Kieselgel, EE/Heptan 4:1) = 0.19. MS (FAB): m/z = 464 $(M+H)^+$.

b) 2-Allyl-5-(2-(5-Chlor-2-methoxy-benzamido)-ethyl)-benzolsulfonamid
Die Herstellung erfolgte aus der Verbindung des Beispiels 18a) nach dem in Beispiel 1e) angeführten Verfahren. Aus 600 mg (1.30 mmol) der Verbindung des Beispiels 18a) resultierten 460 mg der Titelverbindung als hellbrauner Feststoff. Fp: 186 - 187 °C. $R_f$ (Kieselgel, EE/Heptan 4:1) = 0.40. MS (DCI): m/z = 409 $(M+H)^+$.

c) 2-Allyl-5-(2-(5-chlor-2-methoxy-benzamido)-ethyl)-N-(methylaminothiocarbonyl)-benzolsulfonamid
Die Herstellung erfolgte aus der Verbindung des Beispiels 18b) nach dem in Beispiel 1 f) angeführten Verfahren. Aus 150 mg (0.37 mmol) der Verbindung des Beispiels 18b) resultierten nach chromatographischer Reinigung an Kieselgel mit DCM/EE (4:1 ) als Laufmittel 137 mg der Titelverbindung als blaßgelber Feststoff. Fp: 164 - 165 °C. $R_f$ (Kieselgel, EE/Heptan 4:1 ) = 0.19. MS (DCI): m/z = 482 $(M+H)^+$.

d) 2-Allyl-5-(2-(5-chlor-2-methoxy-benzamido)-ethyl)-N-(methylaminocarbonyl)-benzolsulfonamid
Die Herstellung erfolgte aus der Verbindung des Beispiels 18c) nach dem in Beispiel 2) angeführten Verfahren. Aus 68 mg (0.14 mmol) der Verbindung des Beispiels 18c) resultierten 55 mg der Titelverbindung in Form eines weißen Feststoffs. Fp: 124 - 126 °C. $R_f$ (Kieselgel, EE/Heptan 4:1 ) = 0.12. MS (DCI): m/z = 466 $(M+H)^+$.

Beispiel 19

5-(2-(5-Chlor-2-methoxy-benzamido)-ethyl)-2-ethinyl-N-(methylaminothiocarbonyl)-benzolsulfonamid

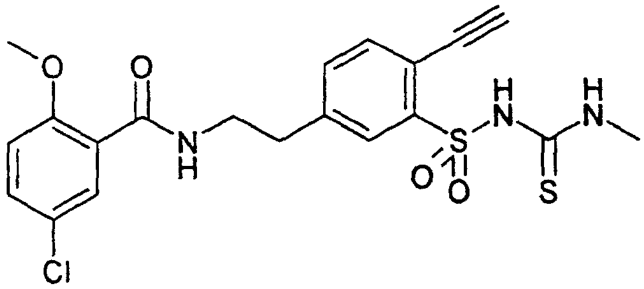

a) 5-(2-(5-Chlor-2-methoxy-benzamido)-ethyl)-N-(dimethylaminomethylen)-2-ethinylbenzolsulfonamid
Die Herstellung erfolgte durch Umsetzung der Verbindung des Beispiels 14e) mit Ethinyl-tributylstannan nach dem in Beispiel 14f) angeführten Verfahren. Aus 800 mg (1.46 mmol) der Verbindung des Beispiels 14e) und 519 mg (1.65 mmol) Ethinyl-tributylstannan wurden nach chromatographischer Reinigung an Kieselgel mit EE/Heptan (4: 1) als Laufmittel 210 mg der Titelverbindung erhalten. $R_f$ (Kieselgel, EE/Heptan 4:1) = 0.10. MS (FAB): m/z = 448 $(M+H)^+$.

b) 5-(2-(5-Chlor-2-methoxy-benzamido)-ethyl)-2-ethinyl-benzolsulfonamid
Die Herstellung erfolgte aus der Verbindung des Beispiels 19a) nach dem in Beispiel 1e) angeführten Verfahren. Aus 210 mg (0.47 mmol) der Verbindung des Beispiels 19a) resultierten 87 mg der Titelverbindung als amorpher Feststoff. $R_f$ (Kieselgel, EE/Heptan 4:1) = 0.23. MS (DCI): m/z = 393 $(M+H)^+$.

c) 5-(2-(5-Chlor-2-methoxy-benzamido)-ethyl)-2-ethinyl-N-(methylaminothiocarbonyl)-benzolsulfonamid
Die Herstellung erfolgte aus der Verbindung des Beispiels 19b) nach dem in Beispiel 1f) angeführten Verfahren. Aus 87 mg (0.22 mmol) der Verbindung des Beispiels 19b) resultierten 47 mg der Titelverbindung als weißer Feststoff. Fp: 224 - 225 °C. $R_f$ (Kieselgel, DCM/EE 4:1) = 0.18. MS (FAB): m/z = 466 $(M+H)^+$.

Beispiel 20

5-(2-(5-Chlor-2-methoxy-benzamido)-ethyl)-N-(methylaminothiocarbonyl)-2-(phenylsulfanyl)-benzolsulfonamid

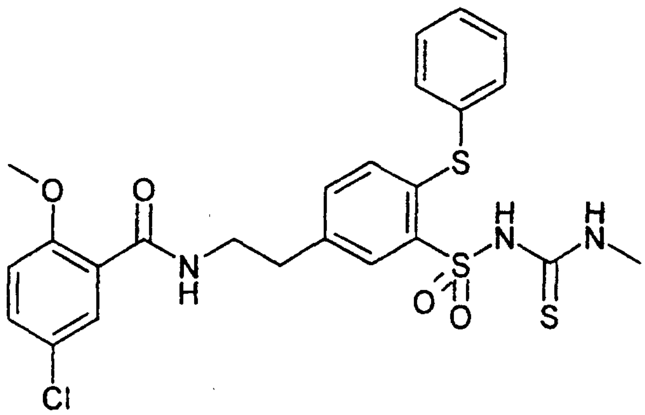

a) 5-(2-(5-Chlor-2-methoxy-benzamido)-ethyl)-N-(dimethylaminomethylen)-2-(phenylsulfanyl)-benzolsulfonamid
Unter einer Argon-Atmosphäre wurden zu einer Suspension aus 493 mg (3.73 mmol) Thiophenol-Natriumsalz und 1.17 g (3.1 mmol) Kupfer(I)iodid in 10.3 ml Hexamethylphosphorsäuretriamid portionsweise 1.7 g (3.1 mmol) der Verbindung des Beispiels 14e) hinzugefügt. Die Reaktionslösung wurde für 6 h bei 80 °C gerührt. Anschließend wurden 40 ml Wasser zugegeben, mehrfach mit EE extrahiert und die vereinigten Extrakte mit gesättigter Kochsalzlösung gewaschen. Nach Trocknung über Natriumsulfat, Einengung und Reinigung des erhaltenen Rückstandes durch Chromatographie an Kieselgel mit EE/Heptan (8:1) wurden 700 mg der Titelverbindung in Form eines weißen Feststoffs isoliert. Fp: 157 - 158 °C. $R_f$ (Kieselgel, EE/Heptan 8:1 ) = 0.40. MS (FAB): m/z = 532 $(M+H)^+$.

b) 5-(2-(5-Chlor-2-methoxy-benzamido)-ethyl)-2-(phenylsulfanyl)-benzolsuffonamid Die Herstellung erfolgte aus der Verbindung des Beispiels 20b) nach dem in Beispiel 1 e) angeführten Verfahren. Aus 300 mg (0.56 mmol) der Verbindung des Beispiels 20b) resultierten 259 mg der Titelverbindung als weißer Feststoff. Fp: 178 °C. $R_f$ (Kieselgel, EE/Heptan 8:1 ) = 0.69. MS (DCI): m/z = 477 $(M+H)^+$.

c) 5-(2-(5-Chlor-2-methoxy-benzamido)-ethyl)-N-(methylaminothiocarbonyl)-2-(phenylsulfanyl)-benzolsulfonamid
Die Herstellung erfolgte aus der Verbindung des Beispiels 20b) nach dem in Beispiel 1f) angeführten Verfahren. Aus 252 mg (0.53 mmol) der Verbindung des Beispiels 20b) resultierten 283 mg der Titelverbindung als weißer Feststoff. Fp: 154 °C. $R_f$ (Kieselgel, EE/Heptan 20:1) = 0.50. MS (DCI): m/z = 550 $(M+H)^+$.

Pharmakologische Untersuchungen

In den folgenden Modellen wurden die therapeutischen Eigenschaften der Verbindungen der Formel I nachgewiesen.

Test 1: Aktionspotentialdauer am Papillarmuskel des Meerschweinchens

a) Einleitung
ATP-Mangelzustände, wie sie während einer Ischämie in der Herzmuskelzelle beobachtet werden, führen zu einer Verkürzung der Aktionspotentialdauer. Sie gelten als eine der Ursachen für sogenannte Reentry-Arrhythmien, die den plötzlichen Herztod verursachen können. Die Öffnung von ATP-sensitiven Kaliumkanälen durch das Absinken von ATP (Adenosintriphosphat) gilt hierfür als ursächlich.

b) Methode
Zur Messung des Aktionspotentials am Papillarmuskel des Meerschweinchens wurde eine Standard-Mikroelektroden-Technik eingesetzt. Hierfür wurden Meerschweinchen beiderlei Geschlechts durch Schlag auf den Kopf getötet, die Herzen entnommen, die Papillarmuskeln herausgetrennt und in einem Organbad aufgehängt. Das Organbad wurde mit Ringerlösung (136 mmol/l NaCl, 3.3 mmol/l KCl, 2.5 mmol/l $CaCl_2$, 1.2 mmol/l $KH_2PO_4$, 1.1

mmol/l $MgSO_4$, 5.0 mmol/l Glucose, 10.0 mmol/l N-(2-Hydroxyethyl)-piperazin-N'-(2-ethansulfonsäure) (HEPES), pH-Wert eingestellt auf 7.4 mit NaOH) durchspült und mit 100 % Sauerstoff bei einer Temperatur von 37 °C begast. Der Muskel wurde über eine Elektrode mit Rechteckimpulsen von 1 V und 1 ms Dauer und einer Frequenz von 1 Hz angeregt. Das Aktionspotential wurde durch eine intrazellulär eingestochene Glas-Mikroelektrode, die mit 3 mol/l KCl-Lösung gefüllt ist, abgeleitet und registriert. Die zu prüfende Substanz wurde der Ringer-Lösung in einer Konzentration von 2 µmol/l zugesetzt. Das Aktionspotential wurde mit einem Verstärker von Hugo Sachs (March-Hugstetten, Deutschland) verstärkt und mittels eines Computers gespeichert und ausgewertet. Die Dauer des Aktionspotentials wurde bei einem Repolarisierungsgrad von 90 % ($APD_{90}$) bestimmt. Die Aktionspotentialverkürzung wurde durch Zugabe einer Lösung des Kaliumkanalöffners Rilmakalim (HOE 234) (W. Linz, E. Klaus, U. Albus, R. H. A. Becker, D. Mania, H. C. Englert, B. A. Schölkens, Arzneimittelforschung/Drug Research 42 (II) (1992) 1180-1185) hervorgerufen (Rilmakalim-Konzentration 1 µg/ml). 30 Minuten nach der Applikation von Rilmakalim wurde die Aktionspotentialdauer $APD_{90}$ registriert. Dann wurde die Testsubstanz zugegeben und nach weiteren 60 Minuten die wieder verlängerte Aktionspotentialdauer $APD_{90}$ registriert. Die Testsubstanzen wurden als Stammlösungen in Propandiol der Badlösung zugesetzt.

c) Resultate

Es wurden folgende $APD_{90}$-Werte (in Millisekunden) registriert.

| Verbindung | Ausgangswert | + HOE 234, 30 Min. | + HOE 234, 30 Min., dann + Substanz, 60 Min. |
|---|---|---|---|
| Beispiel 1 | 170 | 29 | 134 |
| Beispiel 4 | 165 | 28 | 81 |
| Beispiel 7 | 168 | 24 | 71 |
| Beispiel 13 | 177 | 18 | 72 |
| Beispiel 15 | 171 | 33 | 83 |
| Beispiel 16 | 183 | 32 | 153 |
| Beispiel 20 | 191 | 37 | 139 |

Die gefundenen Werte nach 60 Min. belegen die normalisierende Wirkung der erfindungsgemäßen Substanzen auf eine verkürzte Aktionspotentialdauer.

Test 2: Membranpotential an isolierten β-Zellen

a) Einleitung

Der Wirkmechanismus der blutzuckersenkenden Sulfonylharnstoffe wie zum Beispiel des Glibenclamids ist in groben Zügen aufgeklärt. Zielorgan der Sulfonylharnstoffe ist die β-Zelle des Pankreas, wo sie durch eine Beeinflussung des elektrischen Potentials der Zellmembran eine Freisetzung des blutzuckersenkenden Hormons Insulin herbeiführen. Ein hypoglykämischer Sulfonylharnstoff wie zum Beispiel Glibenclamid bewirkt eine Depolarisation der Zellmembran, die zu einem vermehrten Einstrom von Calciumionen und im Gefolge davon zu einer Insulinfreisetzung führt. Das Ausmaß ΔU dieser Depolarisation der Zellmembran durch die Testsubstanzen wurde an insulinsezemierenden RINm5F-Zellen, einer Pankreastumorzellinie, bestimmt. Die Wirkstärke einer Verbindung in diesem Modell sagt das Ausmaß des blutzuckersenkenden Potentials dieser Verbindung voraus.

b) Methode

Zellkulturen von RINm5F-Zellen: RINm5F-Zellen wurden in RPMI 1640-Kulturmedium (Flow), dem 11 mmol/l Glucose, 10 % (v/v) fötales Kälberserum, 2 mmol/l Glutamin und 50 µg/ml Gentamycin zugesetzt wurden, bei 37 °C kultiviert. Die Zellen wurden alle 2 bis 3 Tage auf Petrischalen ausgesät und in einer befeuchteten Atmosphäre von 95 % $O_2$ und 5 % $CO_2$ bei einer Temperatur von 37 °C gehalten. Für die Untersuchungen wurden die Zellen durch Inkubation (ca. 3 Min.) in einem $Ca^{2+}$-freien Medium, das 0.25 % Trypsin enthält, isoliert.

Meßmethode: Isolierte RINm5F-Zellen wurden in einer Plexiglaskammer auf einem inversen Mikroskop, das mit Differential-Interferenz-Kontrast-Optik ausgerüstet war, aufgebracht. Unter optischer Kontrolle (400-fache Vergrößerung) wurde eine feuerpolierte Mikropipette mit einem Öffnungsdurchmesser von etwa 1 µm mit Hilfe eines Mikromanipulators auf die Zelle aufgesetzt. Durch Anlegen eines leichten Unterdruckes an das Innere der Patch-Pipette wurde zunächst eine hohe elektrische Abdichtung zwischen Glas und Zellmembran hergestellt. Anschließend wurde durch Erhöhung des Unterdruckes der Membranfleck unter der Meßpipette aufgerissen. In dieser Ganzzell-Konfiguration wurde mit Hilfe eines Patch-Clamp-Verstärkers (UM EPC 7, List, Darmstadt) das Zellpotential registriert und durch Anlegen einer Spannungsrampe der Ganzzell-Strom gemessen. Die Patch-Pipette war mit einer KCl-Lösung gefüllt, die 140 mmol/l KCl, 10 mmol/l NaCl, 1.1 mmol/l $MgCl_2$, 0.5 mmol/l EGTA, 1 mmol/l Mg-ATP, 10 mmol/l HEPES enthielt, und die einen pH-Wert von 7.2 hatte. Im Bad befand sich eine NaCl-Lösung, die 140 mmol/l NaCl, 4.7 mmol/l KCl, 1.1 mmol/l $MgCl_2$, 2.0 mmol/l $CaCl_2$, 10 mmol/l HEPES enthielt und die einen pH-Wert von 7.4 hatte. Von den Testsubstanzen wurden Stammlösungen in einer Konzentration von 100 mmol/l in DMSO und entsprechende Verdünnungen in einer NaCl-Lösung hergestellt. DMSO alleine hatte keinen Effekt auf das Zellpotential. Um das Zellpotential zu stabilisieren, wurde Diaxozid (100 µmol/l), ein Öffner für ATP-sensitive $K^+$-Kanäle, bei allen Versuchen der Badlösung zugesetzt. Alle Experimente wurden bei 34 ± 1 °C durchgeführt.

c) Resultate
Folgende Werte ΔU, das heißt durch den Zusatz der Testsubstanzen bewirkte Änderungen (Depolarisierungen) der Zellpotentiale, wurden gemessen. Die angeführten Kontrollwerte sind die Zellpotentiale U vor der Zugabe der Testsubstanzen. Zum Vergleich sind die Werte angegeben, die in diesem Test mit Glibenclamid, einen typischen hypoglykämischen Benzolsulfonylhamstoff, erhalten wurden.

| Verbindung | Konzentration | ΔU Kontrollwert |
|---|---|---|
| Beispiel 1 | 1 µmol/l | 6 mV - 74 mV |
| Beispiel 9 | 1 µmol/l | 15 mV - 74 mV |
| Beispiel 12 | 1 µmol/l | 5 mV - 78 mV |
| Beispiel 14 | 1 µmol/l | 7 mV - 70 mV |
| Glibenclamid | 1 µmol/l | 47 mV - 73 mV |

Die gefundenen Werte belegen, daß die erfindungsgemäßen Substanzen keine oder nur eine geringe hypoglykämische Wirkung haben.

## Patentansprüche

**1.** Verbindungen der Formel I,

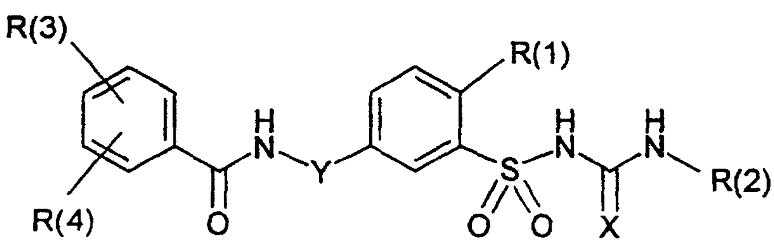

I

in der
X für Sauerstoff oder Schwefel steht;
Y für $-(CR(5)_2)_n-$ steht;
R(1) für

1. Phenyl, das unsubstituiert ist oder substituiert ist durch einen oder zwei gleiche oder verschiedene Substituenten aus der Reihe Halogen, $(C_1-C_4)$-Alkyl, $(C_1-C_4)$-Alkoxy, $-S(O)_m-(C_1-C_4)$-Alkyl, Phenyl, Amino, Hydroxy, Nitro, Trifluormethyl, Cyan, Hydroxycarbonyl, Carbamoyl, $(C_1-C_4)$-Alkoxycarbonyl und Formyl; oder
2. Naphthyl; oder
3. monocyclisches oder bicyclisches Heteroaryl mit ein oder zwei gleichen oder verschiedenen Ring-Heteroatomen aus der Reihe Sauerstoff, Schwefel und Stickstoff; oder
4. $-S(O)_m$-Phenyl; oder

5. ($C_2$-$C_5$)-Alkenyl, das unsubstituiert ist oder substituiert ist durch einen Rest aus der Reihe Phenyl, Cyan, Hydroxycarbonyl und ($C_1$-$C_4$)-Alkoxycarbonyl; oder
6. ($C_2$-$C_5$)-Alkinyl, das unsubstituiert ist oder substituiert ist durch einen Rest aus der Reihe Phenyl und ($C_1$-$C_4$)-Alkoxy;

steht; R(2) für Wasserstoff oder ($C_1$-$C_3$)-Alkyl steht;
R(3) und R(4) unabhängig voneinander für Wasserstoff, Halogen oder ($C_1$-$C_4$)-Alkoxy stehen;
die Reste R(5), die alle unabhängig voneinander sind und gleich oder verschieden sein können, für Wasserstoff oder ($C_1$-$C_3$)-Alkyl stehen;
m für 0, 1 oder 2 steht;
n für 1, 2, 3 oder 4 steht;
in allen ihren stereoisomeren Formen und Mischungen davon in allen Verhältnissen, und ihre physiologisch verträglichen Salze.

**2.** Verbindungen der Formel I gemäß Anspruch 1, worin R(1) für

1. Phenyl, das unsubstituiert ist oder durch einen Substituenten aus der Reihe Halogen, ($C_1$-$C_4$)-Alkyl, ($C_1$-$C_4$)-Alkoxy, -$S(O)_m$-($C_1$-$C_4$)-Alkyl, Trifluormethyl und Nitro substituiert ist; oder
2. monocyclisches Heteroaryl mit ein oder zwei gleichen oder verschiedenen Ring-Heteroatomen aus der Reihe Sauerstoff, Schwefel und Stickstoff; oder
3. -S-Phenyl; oder
4. ($C_2$-$C_3$)-Alkenyl; oder
5. Ethinyl oder 2-Phenylethinyl

steht, in allen ihren stereoisomeren Formen und Mischungen davon in allen Verhältnissen, und ihre physiologisch verträglichen Salze.

**3.** Verbindungen der Formel I gemäß Anspruch 1 und/oder 2, worin R(2) für ($C_1$-$C_3$)-Alkyl steht, in allen ihren stereoisomeren Formen und Mischungen davon in allen Verhältnissen, und ihre physiologisch verträglichen Salze.

**4.** Verbindungen der Formel I gemäß einem oder mehreren der Ansprüche 1 bis 3, worin Y für $-(CH_2)_n-$ steht, in allen ihren stereoisomeren Formen und Mischungen davon in allen Verhältnissen, und ihre physiologisch verträglichen Salze.

**5.** Verbindungen der Formel Ia gemäß einem oder mehreren der Ansprüche 1 bis 4,

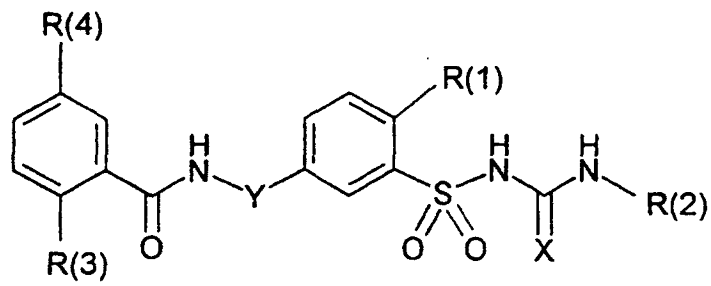

Ia

in der
Y für $-CH_2-CH_2-$ steht;
R(2) für Methyl, Ethyl oder Isopropyl steht;
R(3) für ($C_1$-$C_4$)-Alkoxy steht;
R(4) für Halogen steht;
in allen ihren stereoisomeren Formen und Mischungen davon in allen Verhältnissen, und ihre physiologisch verträglichen Salze.

**6.** Verfahren zur Herstellung von Verbindungen der Formel I gemäß einem oder mehreren der Ansprüche 1 bis 5, **dadurch gekennzeichnet, daß** man eine Verbindung der Formel VIII

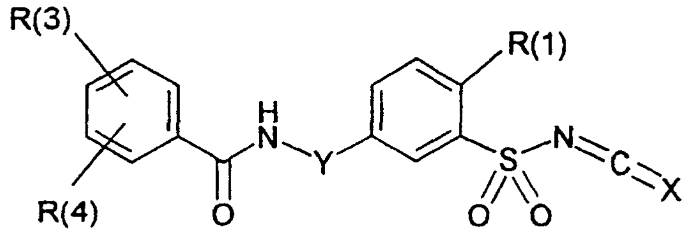

VIII

mit einem Amin der Formel R(2)-$NH_2$ umsetzt, oder daß man zur Herstellung einer Verbindung der Formel I, in der X für Schwefel steht, ein Benzolsulfonamid der Formel III

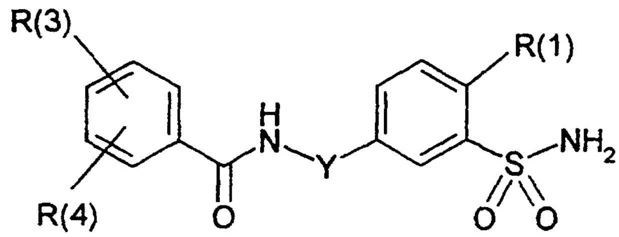

III

oder ein Salz davon mit einem R(2)-substituierten Isothiocyanat der Formel R(2)-N=C=S umsetzt, oder daß man zur Herstellung einer Verbindung der Formel I, in der X für Sauerstoff steht, ein Benzolsulfonamid der Formel III oder ein Salz davon mit einem R(2)-substituierten Isocyanat der Formel R(2)-N=C=O oder mit einem R(2)-substituierten 2,2,2-Trichloracetamid der Formel $Cl_3C$-CO-NH-R(2) umsetzt, oder daß man zur Herstellung einer Verbindung der Formel I, in der X für Sauerstoff steht, die entsprechende Verbindung der Formel I, in der X für Schwefel steht, an der Thioharnstoffgruppe entschwefelt, wobei R(1), R(2), R(3), R(4), X und Y die in den Ansprüchen 1 bis 5 angegebenen Bedeutungen haben.

**7.** Verbindungen der Formel I gemäß einem oder mehreren der Ansprüche 1 bis 5 und/oder ihre physiologisch verträglichen Salze zur Verwendung als Arzneimittel.

**8.** Pharmazeutisches Präparat, **dadurch gekennzeichnet, daß** es eine oder mehrere Verbindungen der Formel I gemäß einem oder mehreren der Ansprüche 1 bis 5 und/oder ihre physiologisch verträglichen Salze und einen pharmazeutisch verträglichen Träger enthält.

**9.** Verbindungen der Formel I gemäß einem oder mehreren der Ansprüche 1 bis 5 und/oder ihre physiologisch verträglichen Salze zur Verwendung als Inhibitoren von ATP-sensitiven Kaliumkanälen.

**10.** Verbindungen der Formel I gemäß einem oder mehreren der Ansprüche 1 bis 5 und/oder ihre physiologisch verträglichen Salze zur Verwendung in der Therapie oder Prophylaxe von cardiovaskulären Erkrankungen, cerebralen Gefäßerkrankungen, ischämischen Zuständen des Herzens, einer geschwächten Herzkraft oder Herzrhythmusstörungen, oder zur Verhinderung des plötzlichen Herztodes oder zur Verbesserung der Herzfunktion nach einer Herztransplantation.

## Claims

**1.** A compound of the formula I

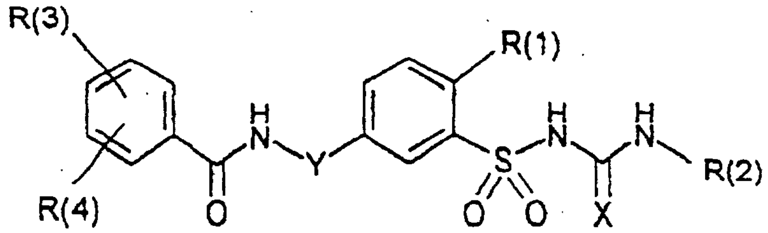

I

in which
X is oxygen or sulfur;
Y is $-(CR(5)_2)_n-$;
R(1) is

1. phenyl which is unsubstituted or is substituted by one or two identical or different substituents from the group consisting of halogen, $(C_1-C_4)$-alkyl, $(C_1-C_4)$-alkoxy, $-S(O)_m-(C_1-C_4)$-alkyl, phenyl, amino, hydroxyl, nitro, trifluoromethyl, cyano, hydroxycarbonyl, carbamoyl, $(C_1-C_4)$-alkoxycarbonyl and formyl; or
2. naphthyl; or
3. monocyclic or bicyclic heteroaryl having one or two identical or different ring heteroatoms from the group consisting of oxygen, sulfur and nitrogen; or
4. $-S(O)_m$-phenyl; or
5. $(C_2-C_5)$-alkenyl which is unsubstituted or is substituted by a radical from the group consisting of phenyl, cyano, hydroxycarbonyl and $(C_1-C_4)$-alkoxycarbonyl; or
6. $(C_2-C_5)$-alkynyl which is unsubstituted or is substituted by a radical from the group consisting of phenyl and $(C_1-C_4)$-alkoxy;

R(2) is hydrogen or $(C_1-C_3)$-alkyl;
R(3) and R(4) independently of one another are hydrogen, halogen or $(C_1-C_4)$-alkoxy;
the radicals R(5), which are all independent of one another and can be identical or different, are hydrogen or $(C_1-C_3)$-alkyl;
m is 0, 1 or 2;
n is 1, 2, 3 or 4;
in all its stereoisomeric forms and mixtures thereof in all ratios, or its physiologically tolerable salts.

**2.** A compound of the formula I as claimed in claim 1, in which R(1) is

1. phenyl which is unsubstituted or is substituted by a substituent from the group consisting of halogen, $(C_1-C_4)$-alkyl, $(C_1-C_4)$-alkoxy, $-S(O)_m-(C_1-C_4)$-alkyl, trifluoromethyl and nitro; or
2. monocyclic heteroaryl having one or two identical or different ring heteroatoms from the group consisting of oxygen, sulfur and nitrogen; or
3. -S-phenyl; or
4. $(C_2-C_3)$-alkenyl; or
5. ethynyl or 2-phenylethynyl

in all its stereoisomeric forms and mixtures thereof in all ratios, or its physiologically tolerable salts.

**3.** A compound of the formula I as claimed in claim 1 and/or 2, in which R(2) is $(C_1-C_3)$-alkyl, in all its stereoisomeric forms and mixtures thereof in all ratios, or its physiologically tolerable salts.

**4.** A compound of the formula I as claimed in one or more of claims 1 to 3, in which Y is $-(CH_2)_n-$, in all its stereoisomeric forms and mixtures thereof in all ratios, or its physiologically tolerable salts.

**5.** A compound of the formula Ia as claimed in one or more of claims 1 to 4.

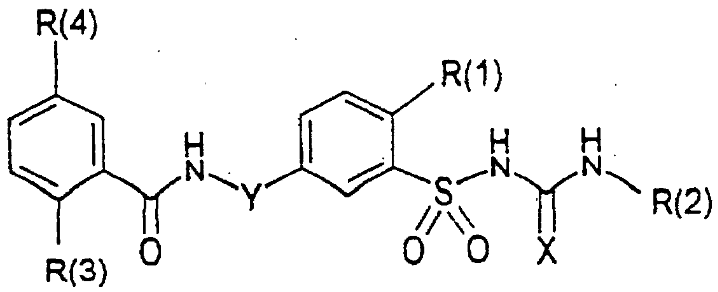

Ia

in which
Y is $-CH_2-CH_2-$;
R(2) is methyl, ethyl or isopropyl;
R(3) is $(C_1-C_4)$-alkoxy;
R(4) is halogen;
in all its stereoisomeric forms and mixtures thereof in all ratios, or its physiologically tolerable salts.

6. A process for the preparation of compounds of the formula I as claimed in one or more of claims 1 to 5, which comprises reacting a compound of formula VIII

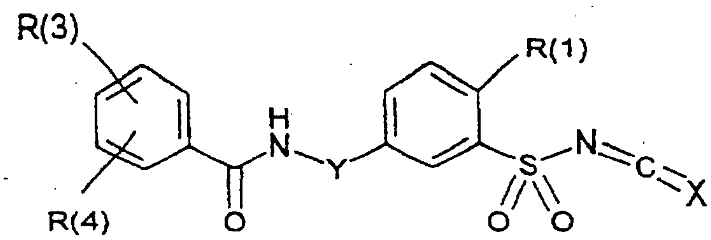

VIII

with an amine of the formula $R(2)-NH_2$ or, for the preparation of a compound of the formula I in which X is sulfur, reacting a benzenesulfonamide of the formula III

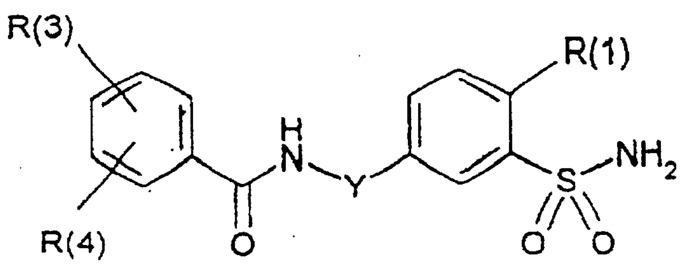

III

or a salt thereof with an R(2)-substituted isothiocyanate of the formula R(2)-N=C=S or, for the preparation of a compound of the formula I in which X is oxygen, reacting a benzenesulfonamide of the formula III or a salt thereof with an R(2)-substituted isocyanate of the formula R(2)-N=C=O or with an R(2)-substituted 2,2,2-trichloroacetamide of the formula $Cl_3C-CO-NH-R(2)$ or, for the preparation of a compound of the formula I in which X is oxygen, desulfurizing the corresponding compound of the formula I in which X is sulfur on the thiourea group, where R(1), R(2), R(3), R(4), X and Y have the meanings indicated in claims 1 to 5.

7. A compound of the formula I as claimed in one or more of claims 1 to 5 and/or its physiologically tolerable salts for use as a medicament.

8. A pharmaceutical preparation, which comprises one or more compounds of the formula I as claimed in one or more of claims 1 to 5 and/or its/their physiologically tolerable salts and a pharmaceutically tolerable carrier.

9. A compound of the formula I as claimed in one or more of claims 1 to 5 and/or its physiologically tolerable salts for use as an inhibitor of ATP-sensitive potassium channels.

10. A compound of the formula I as claimed in one or more of claims 1 to 5 and/or its physiologically tolerable salts for use in the therapy or prophylaxis of cardiovascular disorders, cerebral vascular disorders, ischemic conditions of the heart, a weakened myocardial contractile force or cardiac arrythmias or for the prevention of sudden heart death or for the improvement of the heart function after a heart transplantation.

## Revendications

1. Composés de la formule I :

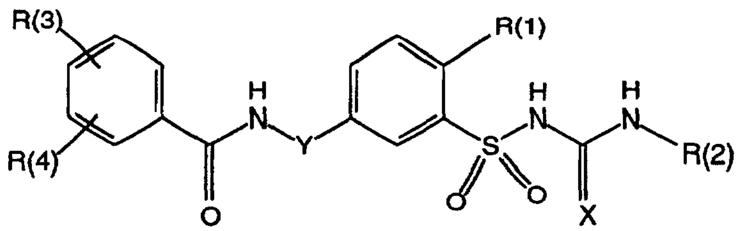

I

dans laquelle
X représente l'atome d'oxygène ou de soufre ;
Y représente $-(CR(5)_2)_n-$ ;
R(1) représente :

1.phényle, qui est non substitué ou substitué par un ou deux substituants identiques ou différents de la série des halogènes, radicaux alkyle ($C_1$-$C_4$), alcoxy ($C_1$-$C_4$), $-S(O)_m$-alkyle ($C_1$-$C_4$), phényle, amino, hydroxyle, nitro, trifluorométhyle, cyano, hydroxycarbonyle, carbamoyle, alcoxy ($C_1$-$C_4$)-carbonyle et formyle, ou
2.naphtyle, ou
3.hétéroaryle monocyclique ou bicyclique avec un ou deux hétéroatomes cycliques identiques ou différents de la série des atomes d'oxygène, de soufre et d'azote, ou
4. $-S(O)_m$-phényle, ou
5.alcényle ($C_2$-$C_5$), qui est non substitué ou substitué par un reste de la série des radicaux phényle, cyano, hydroxycarbonyle et alcoxy ($C_1$-$C_4$)-carbonyle, ou
6.alcynyle ($C_2$-$C_5$), qui est non substitué ou substitué par un reste de la série des radicaux phényle et alcoxy ($C_1$-$C_4$) ;

R(2) représente hydrogène ou alkyle ($C_1$-$C_3$) ;
R(3) et R(4) représentent indépendamment l'un de l'autre, hydrogène, halogène ou alcoxy ($C_1$-$C_4$) ;
les restes R(5), qui sont tous indépendants l'un de l'autre et peuvent être identiques ou différents, représentent hydrogène ou alkyle ($C_1$-$C_3$) ;
m représente 0, 1 ou 2 ;
n représente 1, 2, 3 ou 4 ;
sous toutes les formes stéréoisomères et mélanges de celles-ci en tous rapports, et leurs sels physiologiquement compatibles.

2. Composés de la formule I selon la revendication 1, où R(1) représente :

1.phényle, qui est non substitué ou substitué par un substituant de la série des halogènes, radicaux alkyle ($C_1$-$C_4$), alcoxy ($C_1$-$C_4$), $-S(O)_m$-alkyle ($C_1$-$C_4$), trifluorométhyle et nitro, ou
2.hétéroaryle monocyclique avec un ou deux hétéroatomes cycliques identiques ou différents de la série des

atomes d'oxygène, de soufre et d'azote, ou
3.-S-phényle, ou
4.alcényle ($C_2$-$C_3$) ou
5.éthynyle ou 2-phényléthynyle,

sous toutes les formes stéréoisomères et mélanges de celles-ci en tous rapports, et leurs sels physiologiquement compatibles.

**3.** Composés de la formule I selon les revendications 1 et/ou 2, où R(2) représente alkyle ($C_1$-$C_3$), sous toutes les formes stéréoisomères et mélanges de celles-ci en tous rapports, et leurs sels physiologiquement compatibles.

**4.** Composés de la formule I selon une ou plusieurs des revendications 1 à 3, où Y représente $-(CH_2)_n-$, SOUS toutes les formes stéréoisomères et mélanges de celles-ci en tous rapports, et leurs sels physiologiquement compatibles.

**5.** Composés de la formule la selon une ou plusieurs des revendications 1 à 4 :

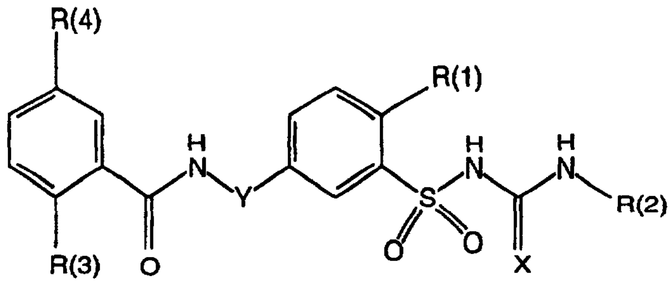

Ia

dans laquelle
Y représente $-CH_2-CH_2-$ ;
R(2) représente méthyle, éthyle ou isopropyle ;
R(3) représente alcoxy en ($C_1$-$C_4$) ;
R(4) représente halogène,
sous toutes les formes stéréoisomères et mélanges de celles-ci en tous rapports, et leurs sels physiologiquement compatibles.

**6.** Procédé de préparation de composés de la formule I selon une ou plusieurs des revendications 1 à 5, **caractérisé en ce que** l'on fait réagir un composé de la formule VIII :

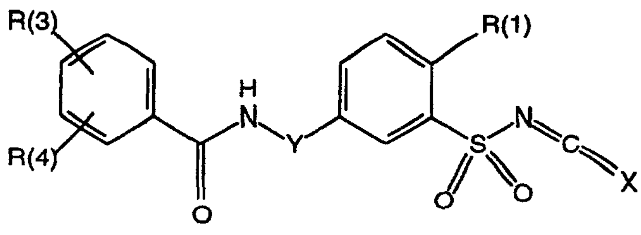

VIII

avec une amine de la formule $R(2)-NH_2$, où **en ce que** l'on fait réagir pour la préparation d'un composé de la formule I dans laquelle X représente soufre, un benzènesulfonamide de la formule III :

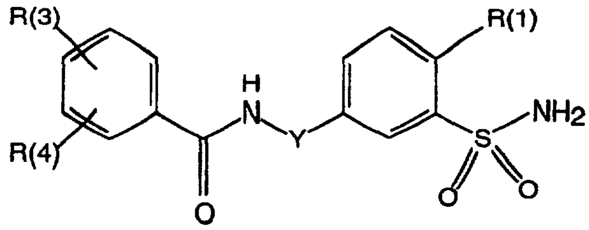

III

ou un sel de celui-ci avec un isothiocyanate substitué par R(2) de la formule R(2)-N=C=S ou **en ce que** l'on fait réagir, pour la préparation d'un composé de la formule I dans laquelle X représente oxygène, un benzènesulfonamide de la formule III ou un sel de celui-ci avec un isocyanate substitué par R(2) de la formule R(2)-N=C=O ou avec un 2,2,2-trichloroacétamide substitué par R(2) de la formule $Cl_3C$-CO-NH-R(2), ou **en ce que** l'on désulfure pour la préparation d'un composé de la formule I dans laquelle X représente oxygène, un composé correspondant de la formule I, dans laquelle X représente soufre, sur le radical thiourée, où R(1), R(2), R(3), R(4), X et Y ont les significations indiquées aux revendications 1 à 5.

**7.** Composés de la formule I selon une ou plusieurs des revendications 1 à 5, et/ou leurs sels physiologiquement compatibles, à utiliser comme médicament.

**8.** Préparation pharmaceutique, **caractérisée en ce qu'**elle contient un ou plusieurs composés de la formule I selon une ou plusieurs des revendications 1 à 5, et/ou leurs sels physiologiquement compatibles, et un support pharmaceutiquement compatible.

**9.** Composés de la formule I selon une ou plusieurs des revendications 1 à 5, et/ou leurs sels physiologiquement compatibles, à utiliser comme inhibiteurs des canaux potassium sensibles à l'ATP.

**10.** Composés de la formule I selon une ou plusieurs des revendications 1 à 5, et/ou leurs sels physiologiquement compatibles, à utiliser dans la thérapie ou la prophylaxie de maladies cardiovasculaires, de maladies vasculaires cérébrales, d'états ischémiques du coeur, d'une puissance cardiaque affaiblie ou des troubles du rythme cardiaque, ou pour empêcher les morts subites par arrêt cardiaque ou pour améliorer la fonction cardiaque après une transplantation de coeur.